(19)

Europäisches
Patentamt
European
Patent Office
Office européen
des brevets

(11) **EP 4 455 277 A1**

(12) **EUROPEAN PATENT APPLICATION**
published in accordance with Art. 153(4) EPC

(43) Date of publication:
**30.10.2024  Bulletin 2024/44**

(21) Application number: **22912059.7**

(22) Date of filing: **26.12.2022**

(51) International Patent Classification (IPC):
**C12N 9/10** (2006.01)     **C12P 19/56** (2006.01)
**C12P 19/18** (2006.01)     **C12N 15/62** (2006.01)
**C12N 15/81** (2006.01)

(52) Cooperative Patent Classification (CPC):
**C12N 9/10; C12N 15/62; C12N 15/81; C12P 19/18; C12P 19/56**

(86) International application number:
**PCT/KR2022/021326**

(87) International publication number:
**WO 2023/121426 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.12.2021  KR 20210187435**

(71) Applicant: **Samyang Corporation**
**Jongno-gu**
**Seoul 03129 (KR)**

(72) Inventors:
• **KO, Hyeokjin**
  **Suwon-si, Gyeonggi-do 16488 (KR)**

• **MOON, Junho**
  **Seongnam-si, Gyeonggi-do 13588 (KR)**
• **KIM, Jeongmin**
  **Yongin-si, Gyeonggi-do 16826 (KR)**
• **KIM, Min Young**
  **Suwon-si, Gyeonggi-do 16577 (KR)**
• **PARK, Busoo**
  **Hanam-si, Gyeonggi-do 13014 (KR)**

(74) Representative: **Schön, Christoph**
**Dr. Schön, Neymeyr & Partner mbB**
**Bavariaring 26**
**80336 München (DE)**

Remarks:
The complete document including Reference Table(s) and the Sequence Listing(s) can be downloaded from the EPO website

(54) **PRODUCTION OF REBAUDIOSIDE**

(57)     The present disclosure relates to a composition for producing steviol glycosides containing Rebaudioside M at high purity, using a biocatalyst containing UDP-glycosyltransferase and UGT76G1, and a method for producing steviol glycosides using the same.

EP 4 455 277 A1

【FIG. 2】

**Description**

[TECHNICAL FIELD]

The present invention relates to production of rebaudioside using a biocatalyst reaction, for example, a microorganism for producing rebaudioside and a method for producing rebaudioside using the same, and more specifically, a recombinant microorganism for producing rebaudioside including UDP-glycosyltransferase, and a method of producing rebaudioside from stevia extract at a high concentration using the microorganisms.

In addition, the present invention also relates to an improved method for producing rebaudioside, particularly a microorganism for producing rebaudioside using freeze-dried cells that stably maintain enzyme activity for a long period of time, and a method for producing rebaudioside using the same.

[RELATED ART]

Sweeteners are known to be the most commonly used ingredients in the food, beverage, or confectionery industries. Sweeteners can be incorporated into the final food product during the food production process, or can be used alone as a table sweetener or as a household substitute for sugar in baking, when they are diluted appropriately. Sweeteners include natural sweeteners such as sucrose, high fructose corn syrup, molasses, maple syrup, and honey, and synthetic sweeteners such as aspartame, saccharin, and sucralose.

Stevia extract is a natural sweetener that can be extracted from the perennial shrub, Stevia rebaudiana. Stevia extracts, purified to various degrees, are used as high-intensity seasonings in foods and blends or are sold alone as table sweeteners.

Extracts of the stevia plant contain rebaudioside and other steviol glycosides that contribute to its sweet taste, but conventional commercial products are primarily Rebaudioside A, and includes at a smaller amounts of other glycosides (glycosides) such as Rebaudioside C, D, and F. Stevia extracts derived from the plant may contain contaminants such as derived compounds causing off-flavors. The off-flavors can be problematic depending on the food system or application chosen.

Additionally, the composition of stevia extract varies greatly depending on the soil and climate in which the plant grows. Depending on the source plant, climatic conditions, and extraction process, the content of Rebaudioside A obtained from commercial manufacturing process is reported to vary from 20 to 97% of the total content of steviol glycosides. Other steviol glycosides are present in varying amounts in stevia extract.

However, stevia extract produced from the stevia plant contains several steviol glycosides and compounds that cause off-flavors, and recovery and purification are labor-intensive and inefficient. Accordingly, there is a need for a production system that can produce and accumulate steviol glycosides such as Reb D and Reb M at high yield.

In particular, Reb M is 200 to 350 times sweeter than sugar and has a clean sweet taste with a slightly bitter or licorice-like aftertaste. Recently, the EFSA Panel on Food Additives and Flavorings announced that Reb M is safe as a food additive. Because Reb M is suitable for blending and applicable to various food and beverage products, Reb M may attract great interest and generate continued demand as a natural sweetener in the global food industry.

Furthermore, there is an urgent need for a biocatalyst having excellent storage and distribution properties that maintains conversion activity and for a mass production process for steviol glycosides, especially Rebaudioside M, using a biocatalyst, by establishing a manufacturing process and conditions to remove moisture contained in a biocatalyst such as a microbial cell that produces an enzyme with conversion activity of steviol glycosides.

[DISCLOSURE]

[TECHNICAL PROBLEM]

An embodiment of the present invention relates to a composition for producing steviol glycosides which comprises a biocatalyst containing a first uridine diphosphate-glucosyltransferase (UDP-glucosyltransferase) having an amino acid sequence identity of more than 92% with the amino acid sequence of SEQ ID NO: 1 and transferring glucose to the steviol glycoside substrate and UGT76G1, and a reacting substrate; or a production method of steviol glycosides, in which Rebaudioside M is contained at 10 % by weight or higher in a reaction product of steviol glycoside.

An embodiment of the present invention is to provide a composition for producing Rebaudioside M with high purity comprising a biocatalyst which includes a recombinant microorganism for producing rebaudioside having UDP-glycosyltransferase derived from *Triticum aestivum,* preferably the UDP-glycosyltransferase and UDP-glycosyltransferase 76G1, or more preferably the UDP-glycosyltransferase, UDP-glycosyltransferase 76G1 and sucrose synthase.

An embodiment of the present invention is to provide a composition for producing steviol glycosides including Rebaudioside M with high purity, comprising a biocatalyst which includes a recombinant microorganism for producing rebaudioside having UDP-glycosyltransferase derived from *Triticum aestivum,* preferably the UDP-glycosyltransferase and UDP-glycosyltransferase 76G1, or more preferably the UDP-glycosyltransferase, UDP-glycosyltransferase 76G1 and sucrose synthase, in which Rebaudioside M is contained at 50 % by weight or higher in a reaction product of steviol glycoside.

A further embodiment of the present invention is to provides a method of producing steviol glycosides containing Rebaudioside M, including a step of reacting a reacting substrate containing at least one selected from the group consisting of stevioside and Rebaudioside A, with a biocatalyst which includes UDP-glycosyltransferase derived from *Triticum aestivum,* and UGT76G1.

A further embodiment of the present invention relates to a microorganism for producing rebaudioside using freeze-dried microbial cells that can stably maintain the enzyme activity of the microorganism for a long period of time in a dry state, and a method for producing rebaudioside using the same.

A further embodiment of the present invention may be microbial cells or dried cells thereof having the converting activity of steviol glycosides, especially Rebaudioside M.

A further embodiment of the present invention provides a composition for producing steviol glycosides, especially Rebaudioside M, or a method of producing steviol glycosides, especially Rebaudioside M, comprising microbial cells or dried cells thereof having conversion activity of steviol glycosides, particularly Rebaudioside M.

[TECHNICAL SOLUTION]

The present disclosure describes a composition for producing Rebaudioside M with high purity, using a biocatalyst which includes a recombinant microorganism for producing rebaudioside having UDP-glycosyltransferase derived from *Triticum aestivum,* preferably the UDP-glycosyltransferase and UDP-glycosyltransferase 76G1, or more preferably the UDP-glycosyltransferase, UDP-glycosyltransferase 76G1 and sucrose synthase, and or a method of producing steviol glycosides using the same.

An embodiment of the present invention relates to a composition for producing steviol glycosides which comprises a biocatalyst containing a first uridine diphosphate-glucosyltransferase (UDP-glucosyltransferase) derived from *Triticum aestivum,* particularly having an amino acid sequence identity of 92% or higher with the amino acid sequence of SEQ ID NO: 1 and transferring glucose to the steviol glycoside substrate and UGT76G1, and a reacting substrate, in which Rebaudioside M is contained at 50 % by weight or higher in a reaction product of steviol glycoside.

Additional embodiment of the present invention relates to a composition for producing steviol glycosides which comprises a biocatalyst containing a first uridine diphosphate-glucosyltransferase (UDP-glucosyltransferase) having an amino acid sequence identity of 92% or higher with the amino acid sequence of SEQ ID NO: 1 and transferring glucose to steviol glycoside substrate and UGT76G1, and a reacting substrate, in which the biocatalyst is at least one selected from the group consisting of cells of microorganisms producing the enzyme protein, and dried products of the cells, and in which Rebaudioside M is contained at 10 % by weight or higher in a reaction product of steviol glycoside.

In addition, when performing a conversion reaction using a composition for producing steviol glycosides containing Rebaudioside (Reb) M, for example, when using a raw material substrate containing Reb A and stevioside, Reb M is contained at 10 % by weight or more, 15% by weight or more, 20% by weight or more, 30% by weight or more, 40% by weight or more, 50% by weight or more, 60% by weight or more, 70% by weight or more, 80% by weight or more, 85% by weight or more, 86% by weight. % or more, 87% by weight or more, or 90% by weight or more in a reaction product of steviol glycoside. The composition for producing steviol glycosides containing Reb M, may have a sum of conversion rate of Reb M and conversion rate of Reb D of 30% by weight or more, 35% by weight or more, 40% by weight or more, 45% by weight or more, or 50% by weight or more.

The composition may preferably further include ethylenediaminetetraacetic acid (EDTA), for example, at an amount of 1 to 20mM, 3 to 20mM, 4 to 20mM, 1 to 15mM, 3 to 15mM, 4 to 15mM, 1 to 10mM, 3 to 10mM, or 4 to 10mM. When a conversion reaction is performed using the composition for producing steviol glycosides containing Reb M includes EDTA, with substrate such as raw materials containing Reb A and stevioside, Reb M is contained at 50% by weight or more, 60% by weight or more, 70% by weight or more, 80% by weight or more, 85% by weight or more, 86% by weight. % or more, 87% by weight or more, or 90% by weight or more in a reaction product of steviol glycoside.

The composition may further include raw reacting materials including at least one selected from the group consisting of stevia extract, uridine diphosphate glucose (UDP), and sucrose. In an embodiment, for performing conversion of Reb A to Reb D, the composition for producing steviol glycosides may further include $MgCl_2$ and/or $MnCl_2$. Another embodiment of the present application is a method for producing steviol glycosides containing Reb M, including a step of reacting a reaction substrate containing at least one selected from the group consisting of stevioside and Rebaudioside A, with a biocatalyst containing a first uridine diphosphate-glucosyltransferase from *Triticum aestivum,* and UGT76G1, in which the first uridine diphosphate-glucosyltransferase has an amino acid sequence identity of more than 92% with the amino acid sequence of SEQ ID NO: 1, and is an enzyme protein (uridine diphosphate-glucosyltransferase) having UDP-glycosyl transferase activity that transfers glucose to a steviol glycoside substrate.

An embodiment of the present invention relates to a method for producing Reb M, a steviol glycoside, comprising: a step of preparing steviol glycosides selected from the group consisting of Reb D and Reb E, by reacting at least one selected from the group consisting of a first UDP-glucosyltransferase protein, a recombinant microorganism expressing the enzyme protein, a cell of the microorganism, a lysate of the microorganism, and a culture of the microorganism. and extracts thereof, with at least one substrate selected from the group consisting of stevioside and Reb A in the presence of a glucose donor, and

a step of reacting the prepared steviol glycosides, with a second UDP-glucosyltransferase protein, a recombinant microorganism expressing the enzyme protein, cells of the microorganism, cell lysates of the microorganism, cultures of the microorganism, and extracts thereof in the presence of glucose donor.

An embodiment of the present invention relates to a method for producing Reb M, a steviol glycoside, comprising a step of reacting at least one substrate selected from the group consisting of stevioside and Reb A, with a biocatalyst containing a first uridine diphosphate-glucosyltransferase from *Triticum aestivum,* and UGT76G1,

in which the first uridine diphosphate-glucosyltransferase has an amino acid sequence identity of 92% or higher with the amino acid sequence of SEQ ID NO: 1, and is an enzyme protein (uridine diphosphate-glucosyltransferase) having UDP-glycosyl transferase activity that transfers glucose to a steviol glycoside substrate, and

in which the biocatalyst is at least one selected from the group consisting of cells of microorganisms that produce the enzyme protein, and dried products of the cells.

The second UDP-glucosyltransferase protein used in the production step of Reb M may be UGT76G1 or the like.

The method for producing steviol glycosides according to the present invention can be economically used in the food and beverage industries because it can significantly shorten the production cycle, improve production capacity, and provide products with lower costs and higher purity.

In steviol glycosides containing Reb M, which is the reaction product in the present invention, the purity of Reb M may be 50% by weight or more.

The reaction substrate may include at least one steviol glycosides selected from the group consisting of stevioside and Reb A, or may be provided in an amount of 15 to 200 g/L. The reaction substrate may further include at least one selected from the group consisting of steviol monoside, nibusoside, Reb E, Reb C, Rebaudioside I, stevioside, Reb D, synthetic steviol glycoside, and combinations thereof.

The diversity of steviol glycosides in S. *rebaudiana* is caused by glycosylation reaction catalyzed by UDP-glycosyltransferases (UGT) which transfer saccharide residues to acceptor molecules from activated donor, mainly uridine diphosphate (UDP)-glucose. Reb M is a preferred natural non-caloric sweetener having high potency which is contained in very low amounts in the leaves of *Stevia rebaudiana.* According to the composition for producing steviol glycosides or the method for producing steviol glycosides according to the present invention, Reb M can be produced with high purity and high conversion rate.

Hereinafter, the present invention will be described in more detail.

The composition for producing steviol glycosides or the method for producing steviol glycosides according to the present invention can use a biocatalyst containing a novel first uridine diphosphate-glucosyltransferase and UGT76G1 as a second UDP-glycosyltransferase. The biocatalyst may further include "sucrose synthase" and may be included as a fusion protein with the first uridine diphosphate-glucosyltransferase and/or UDP-glycosyltransferase.

In another embodiment, a third UDP-glucosyltransferase which is an enzyme capable of adding at least one glucose unit to Reb A to form Reb D, for example UDP-glucosyltransferase UGT91 D2, may be included.

In the present disclosure, the biocatalyst containing the enzyme may include at least one selected from the group consisting of the enzyme protein, the cells of the microorganism, a culture of the microorganism, a lysate of the microorganism, and an extract of the lysate or culture.

In this disclosure, the first uridine diphosphate-glucosyltransferase is an enzyme protein (uridine diphosphate-glucosyltransferase) that has 92% or higher of amino acid sequence identity with the amino acid sequence of SEQ ID NO: 1 and has UDP-glycosyl transfer activity to transfer glucose to a steviol glycoside substrate.

The first uridine diphosphate-glucosyltransferase can convert stevioside to Reb E and/or convert Reb A to Red D, so as to produce Reb D and/or Reb E.

Specifically, the first UDP-glucosyltransferase according to the present invention has a conversion activity of preparing steviol glycosides, for example at least one selected from the group consisting of Reb D and Reb E, from one or more substrates selected from the group consisting of stevioside and Reb A to steviol glycosides, in the presence of a glucose donor, or more specifically can convert Reb A to Reb D and stevioside to Reb E, in which the enzyme has conversion activity for both single or mixed substrates of Reb A and stevioside.

In addition, the conversion from Reb A to Reb D involves transferring glucose through the bonding of the beta 1-2 glyosidic bond in the steviol ring, and glycosylation mainly occurs at position 19 of the steviol ring. Steviol glycosides may be composed of 1 to 3 glucose linked through β bonds.

The conversion reaction from Reb A to Reb D is a reaction in which one glucose is added by UGT enzyme. The glucose required for this reaction is UDP-glucose, which is in its active form, and UGT enzyme transfers glucose from UDP-glucose to produce steviol glycosides.

The first UDP-glucosyltransferase may be an enzyme derived from *Triticum aestivum* (wheat) (*Triticum aestivum* UDP-glycosyltransferase) (hereinafter referred to as TaUGT) or a mutant enzyme thereof (hereinafter referred to as TaUGTm). Specifically, the first UDP-glucosyltransferase is an enzyme protein that has 92% or higher of amino acid sequence identity with the amino acid sequence of SEQ ID NO: 1 and has UDP-glycosyl transfer activity to transfer glucose to a steviol glycoside substrate. An enzyme protein having an amino acid sequence of SEQ ID NO: 1 may be a wild-type enzyme.

The nucleotide sequence of wild-type TaUGT of the present invention may be a nucleotide sequence encoding the amino acid sequence of SEQ ID NO: 1, and may specifically be the nucleotide sequence of SEQ ID NO: 2, but is not limited thereto.

The first UDP-glucosyltransferase may include an amino acid sequence having a sequence identity of at least 92%, at least 95%, at least 97%, at least 99%, or at least 99.5% with the amino acid sequence of SEQ ID NO: 1, and it also includes variant enzyme proteins containing amino acid sequences in which some sequences are deleted, modified, substituted, or added, if it has an activity corresponding to the enzyme protein.

It may be encoded by a polynucleotide sequence having the sequence identity with the polynucleotide sequence encoding the first UDP-glucosyltransferase is 40% or more, 50% or more, 60% or more, 70% or more, 80% or more, 90% or more, 95% or more, 97% or more, or 99% or more.

In the above, the term "homology" or "identity" refers to the degree of matching with a given amino acid sequence or nucleotide sequence and can be expressed as a percentage. Herein, homologous sequence having the same or similar activity corresponding to a given amino acid sequence or nucleotide sequence are expressed as "% homology" or "% identity."

The variant of first UDP-glucosyltransferase has an activity of 101% or higher, for example, 101 % to 200%, based on 100% of the activity of converting the Reb A substrate to Reb D of the wild-type enzyme containing the amino acid sequence of SEQ ID NO: 1. In other words, the production rate of Reb D and Reb E was improved by modifying the enzyme through mutation in order to improve the activity of the enzyme, and can be reacted with a second UDP-glucosyltransferase, for example, UGT76G1 from stevia, to produce Reb M. Accordingly, the productivity of Reb D and Reb E can be increased through an enzymatic conversion reaction, and using this, a highly functional sugar called Reb M can be produced with high productivity.

The variant of first UDP-glucosyltransferase can contain a substitution of serine for threonine at 201$^{st}$ position, a substitution of leucine for valine at 202$^{nd}$ position, or both of a substitution of serine for threonine at 201$^{st}$ position and a substitution of leucine for valine at 202$^{nd}$ position from N-terminus of an amino acid sequence of SEQ ID NO:1, and more specifically, it may include the amino acid sequence of SEQ ID NO: 3.

In addition, the nucleotide sequence encoding the mutant enzyme can include a nucleotide sequence encoding an amino acid sequence containing a substitution of serine for threonine at 201$^{st}$ position, a substitution of leucine for valine at 202$^{nd}$ position, or both of a substitution of serine for threonine at 201$^{st}$ position and a substitution of leucine for valine at 202$^{nd}$ position from N-terminus of an amino acid sequence of SEQ ID NO:1, and more specifically, an amino acid sequence of SEQ ID NO: 3. The mutant enzyme containing an amino acid sequence of SEQ ID NO: 3, or a nucleotide sequence encoding the nucleotide sequence encoding the amino acid sequence may be the nucleotide sequence of SEQ ID NO: 3.

In the mutant enzyme of TaUGTm (T201 S) according to the present invention, Thr at position 201 from the N-terminus of amino acid sequence of SEQ ID NO: 1 of the wild-type enzyme is substituted with Ser, for example, ACC is replaced with TCA. In TaUGTm (V202L), Val at position 201 is substituted with Leu, for example, GTA is substituted with TTA. In TaUGTm (T201S/V202L), Thr-Val may be replaced with Ser-Leu, and more specifically, ACCGTA may be replaced with TCATTA at amino acids 201 and 202 from the N-terminus in the wild-type enzyme amino acid sequence of SEQ ID NO: 1, and more specifically, ACCGTA may be replaced with TCATTA in the polynucleotide sequence of SEQ ID NO: 2.

The variant first UDP-glucosyltransferase according to the present invention has an conversion activity of 45% by weight or more of Reb A into Reb D in an enzymatic reaction for 2 to 24 hours, or specifically, an conversion activity of 45% by weight or more, 50% by weight or more, 70% by weight or more, 75% by weight or more, 80% by weight or more, 85% by weight or more, or 95% by weight or more of Reb A to Reb D, or more preferably, an conversion activity of 70 % by weight or more, 75% by weight or more, 80% by weight or more, 85% by weight or more, or 95% by weight or more of Reb A to Reb D

More specifically, the conversion activity of the enzyme may be measured under conditions of 30°C, pH 7.2, and 150 rpm. The Reb D conversion rate (%) is calculated by Formula 1 below, and more details are described in Example 4.

【Formula 1】

Conversion rate of Reb D (%) = HPLC peak area of Reb D /total HPLC peak area

The variant of first UDP-glucosyltransferase according to the present invention, compared to the wild-type enzyme having an amino acid sequence of SEQ ID NO: 1, has 101% or more, 103% or more, 105% or more, 107% or more, 108% or more, 109% or more, or 110% or more, or more preferably 105% or more, 107% or more, 108% or more, or 109% or more, 110% or more, 115% or more, 120% or more, or 125% or more of a relative conversion rate of Reb D which is an activity of converting Reb A substrate to Reb D in an enzymatic reaction for 2 to 24 hours. The conversion activity of the enzyme may be measured at 30°C, pH 7.2, and 150 rpm. The Reb D conversion rate of the enzyme, which converts to Red D from Reb A as single substrate, is calculated according to Formula 1 below. Specific measurement conditions are described in Example 4 below.

In addition, the variant of first UDP-glucosyltransferase has an activity (sum of Reb D/E conversion rates) of converting 50 % by weight or more, 60 % by weight or more, 70 % by weight or more, 79 % by weight or more, 80 % by weight or more, 81 % by weight or more, 82 % by weight or more, 83 % by weight or more, 85 % by weight or more, or 87 % by weight or more, into Reb D and Reb E in an enzymatic reaction for 2 to 24 hours in mixed substrates containing stevioside and Reb A. More specifically, the conversion activity of the enzyme may be measured under conditions of 30°C, pH 7.2, and 150 rpm.

The variant of first UDP-glucosyltransferase according to the present invention, compared to the wild-type enzyme having an amino acid sequence of SEQ ID NO: 1, has 103% or more, 105% or more, 106% or more, 108% or more, 109% or more, or 110% or more, or more preferably 105% or more, 107% or more, 108% or more, or 109% or more, 110% or more of sum of Reb D/E relative conversion rates which is an activity (sum of Reb D/E conversion rates) of converting 50 % by weight of mixed substrates containing stevioside and Reb A into Reb D and Reb E in an enzymatic reaction for 2 to 24 hours for the mixed substrates containing stevioside and Reb A. More specifically, the conversion activity of the enzyme may be measured using mixed substrates containing Reb A: stevioside = 40:60 under conditions of 30°C, pH 7.2, and 150 rpm. The total Reb D/E relative conversion rate of the enzyme refers to the total Reb D/E conversion rate of the enzyme, based on 100% of the total Reb D/E conversion rate of the enzyme. In other words, the total conversion rate of Reb D/E of the enzyme for converting the mixed substrate to Reb D/E is calculated according to Formula 2 below. The relative total conversion rate of Reb D/E of the enzyme may be the total conversion rate of Reb D/E for the variant TaUGT enzyme, based on 100% of total conversion rates of Reb D/E for the wild type enzyme.

Reb D/E conversion rate (%) = HPLC peak area of Reb D/E / (total HPLC peak area)                [Formula 2]

The wild-type enzyme of first UDP-glucosyltransferase according to the present invention may be an enzyme derived from *Triticum aestivum,* which is obtained by searching for plant-driven enzymes. By identifying the protein function, the enzyme has UGT enzyme activity, and the enzyme activity of converting stevioside or Reb A into Reb E or Reb D, respectively.

Reb D with high sweetness is a saccharide in small amounts in the stevia plant and is produced from Reb A, in which the known enzyme involved in this production process is UGT91D2 of the stevia plant. An enzyme with similar activity is EUGT11 enzyme present in rice. Because UGT91D2 of the stevia plant has an activity of converting Reb A of a steviol glycoside, to Reb D at low conversion activity, it difficult to produce high concentration of Reb M in high yield.

In addition, conventionally known UGT enzymes include UGT (hereinafter referred to as SrUGT) derived from the stevia plant *(Stevia rebaudiana),* UGT (hereinafter referred to as EUGT11) derived from rice *(Oryza sativa),* and UGT (hereinafter referred to as HvUGT) derived from barley *(Hordeum vulgare).* The UDP-glucosyltransferase of the present invention has advantages of substrate specificity that acts on several substrates of steviol glycosides and no production of reaction by-products, compared to the conventionally known EUGT11 and HvUGT. The amino acid sequence of EUGT11 enzyme is shown in SEQ ID NO: 12, and the polynucleotide sequence is shown in SEQ ID NO: 13. The amino acid sequence of HvUGT enzyme is shown in SEQ ID NO: 14, and the polynucleotide sequence is shown in SEQ ID NO: 15.

The wild-type enzyme having the amino acid sequence of SEQ ID NO: 1 had 91% of amino acid sequence identity with HvUGT and 66% with EUGT as a result of analyzing sequence identity with HvUGT and EUGT11, and a polynucleotide sequence identity of 36% with HvUGT and 37% with EUGT11 as a result of analyzing polynucleotide sequence identity.

The composition for producing steviol glycosides or the method for producing steviol glycosides according to the present invention can use a biocatalyst containing UGT76G1 as a second UDP-glycosyltransferase, together with the first uridine diphosphate-glucosyltransferase. SrUGT76G1 of the present invention is a type of SrUGT and is an enzyme with a family number of 76G1. SrUGT76G1 may have an amino acid sequence of SEQ ID NO: 31, but is not limited thereto. The nucleotide sequence of SrUGT76G1 may be a nucleotide sequence encoding an amino acid sequence of SEQ ID NO: 31, and may specifically be the nucleotide sequence of SEQ ID NO: 32, but is not limited thereto.

In the present invention, the second UDP-glycosyltransferase, UGT76G1, is UDP-glycosyltransferase UGT76G1 (Stevia glycosyltransferase UGT76G1), which is a main enzyme responsible for Reb M production in S. *rebaudiana,* and is reported as only enzyme of Reb M biosynthesis from Reb D. UGT76G1 is known to have broad substrate specificity, and specifically, was found to catalyze eight different reactions in the biosynthesis of steviol glycosides, all of which include 1,3-glucosylation of glucose connected to C13 and C19 of steviol. For example, UGT76G1 can convert stevioside into Reb A, Red D into Reb M, and Reb E into Reb M.

SrUGT76G1 has an activity of converting Reb A to Reb I, and can be used together with TaUGTm having an activity of converting Reb E and/or Reb A to Reb D in order to obtain Reb M in high yield from stevia (e.g., stevia extract) and/or Reb A, because Reb I is difficult to convert to Reb M.

The composition for producing steviol glycosides or the method for producing steviol glycosides according to the present invention can use a biocatalyst that further contains Sucrose Synthase (SUS) enzyme in addition to the first uridine diphosphate-glucosyltransferase and the second UDP-glycosyltransferase, UGT76G1.

Sucrose Synthase (SUS) is an enzyme decomposing sucrose to produce fructose and UDP-glucose. Therefore, if UDP-Glucose is supplied to the UGT enzyme using SUS enzyme in the reaction for converting Reb A to Reb D or stevioside to Reb E, the reaction can be carried out more economically by supplying UDP-Glucose. When SUS enzyme is included in the biocatalyst, it is more preferable that the microorganism producing the enzyme is a microorganism of the genus Saccharomyces with deletion of SUC2 gene encoding the invertase enzyme.

The sucrose synthase may be a sucrose synthase derived from one or more microorganisms selected from the group consisting of *Arabidopsis thaliana, Solanum lycopersicum, Glycine max, Nicotiana tabacum,* and *Thermosynechococcus elongates,* but is not limited thereto, and includes all sucrose synthases known in the art can be Applicable to inventions. More specifically, the sucrose synthase may be the SUS1 enzyme derived from *Arabidopsis thaliana,* and may include, for example, an amino acid sequence of SEQ ID NO: 4, and a nucleotide sequence encoding the amino acid sequence of SEQ ID NO: 4, or a nucleotide sequence of SEQ ID NO: 5. In addition, it may be a sucrose synthase derived from Glycine max, and may include, for example, an amino acid sequence of SEQ ID NO: 33, and may have a nucleotide sequence encoding an amino acid sequence of SEQ ID NO: 33 or a nucleotide sequence of SEQ ID NO: 34.

In a specific example, if the conversion reaction is performed by preparing SUS as a fusion protein with UGT, the two reactions can form a single channel so that enzymatic reactions can occur in batches, resulting in an effective conversion reaction. For example, if the conversion reaction is performed by preparing SUS as a fusion protein to UGT, the two reactions can form one channel to perform an enzymatic reaction, resulting in an effective conversion reaction.

In addition, when using the TaUGT_SUS fusion enzyme, it is possible to perform the conversion using 1/10 of UDP amount for the substrate, and using UDP at an amount lower than 1/100 of UDP amount for RA40 substrate. Through the conversion reaction using the fusion enzyme, the substrate conversion can be performed economically and high-concentration substrate reaction can be carried out to produce steviol glycosides.

More specifically, sucrose synthase may be fused directly or through a linker to one or more enzymes selected from the group consisting of the first UDP-glucosyltransferase and the second UDP-glucosyltransferase (e.g., UGT76G1 enzyme) according to the present invention. The linker may be a peptide consisting of 4 to 15 amino acids, or more specifically, 4 to 15 amino acids of at least an amino acid selected from the group consisting of G (Gly), S (Ser) and P (Pro), and may be GGGS (SEQ ID NO: 7), GGGGS (SEQ ID NO: 8), GGGSGGGGS (SEQ ID NO: 9), GGGGSGGGGSGGGGS (SEQ ID NO: 10), or GGGGPSPGGGGS (SEQ ID NO: 11).

In a specific example of the present invention, it may be a fusion protein that will be linked to the first UDP-glucosyltransferase and the SUS1 enzyme derived from *Arabidopsis thaliana* through a linker (GGGGSG), and specifically includes an amino acid sequence of SEQ ID NO: 7, or SEQ ID NO: 7, a nucleotide sequence encoding the amino acid sequence, or a nucleotide sequence of SEQ ID NO: 8.

The specific enzyme sequences described herein are exemplarily shown in Table 1 below, and the mutant enzyme TaUGTm represents UGT (T201S/V202L) in Table 1 below.

[Table 1]

| Name | sequence (5' -> 3') | SEQ ID NO |
|------|---------------------|-----------|
| TaUGT_Protein | MDDGSSSSSSPLRVVICPWLAFGHLLPCLDIAERLASRGHRVSFVS TPRNIARLPPVRPAVAPLVDYVALPLPRVDGLPEGAESTNDVPHDKF ELLRKAFDGLAAPFSEFLHAACAEGTGKRPDWLIVDSFHHWAAAAA VENKVPCVMLLLGAANVIATWARGVSEHAAAAVGKERSAAEAPSFE TERRKLMITQNASGMTVAERYFLTLMRSNLVAIRSCAEWEPESVAAL TTLAGKPVVTLGLLPPSPEGGRGISKQDAAVRWLDAQRDKSVVYVA LGSEVPLRVEQVHELALGLELSGASFLWALRKPPGMPDAAVLPPGF EERTRGRGLVVTGWVPQISVLAHGAVAAFLTHCGWNSTIEGLLFGQ PLIMLPISSDQGPNARLMEGRKVGMQVPRNESDGSFTREDVAATV QAVAMEEDGSRVFTANAKTMQEIVADSACHERCIDGFIQQLRSYKE | 1 |
| TaUGT_DNA | atggacgacgggtcttctagctctagcagtccgctgcgtgttgttatttgcccgtggctggcttttgg acacctgcttccatgcttagacattgcggaaagattagccagccgtggacacagagtatccttc gtaagtacgccgcgtaatatagcacgtttaccccccggtcagaccggcagttgcgccgctggtc gattacgtagcgcacctctgcccagagtggacggacttccggaaggggctgaatctacaaac gacgtgccccatgataagttcgagctttttacgtaaggctttcgacggtttggcagcgcccttcagc gaatttctacacgcagcgtgcgcggagggcacaggcaaaagaccagattggcttattgtggat tccttccaccattgggctgcggctgcggcagtggaaaataaagtcccgtgcgtcatgttactttta ggtgcagccaacgtgatcgccacttgggcacgtggcgtgtcagagcatgcggccgccgcggt tggaaaagaacgtagtgccgcagaagctccatcattcgaaaccgaacgtcgtaaactgatga taactcagaatgcatcaggtatgaccgtagcagagcgttatttcctgacacttatgagatcaaac ttggttgccattcgttcctgtgcagaatgggagcctgaatctgtggcggcactaaccactctggcc gggaagcccgtagtgaccttaggtttgcttccaccgtctcctgagggaggaaggggtatttcca agcaagacgctgccgttaggtggctggacgctcagagggataagtccgttgtatacgtggcgtt agggtctgaagttcccttgagagtggagcaggtacatgagcttgcgcttggactagagttgtcag gtgctagcttcctgtgggcgttacgtaagccacccggcatgcctgacgccgcagtcttgccgcc gggttttgaagagagaaccaggggacgtggtctggttgtgactggctgggtaccacaaatcag cgtcttagcacatggcgccgttgccgctttctgacacattgcggctggaatagtacaatcgaag gcttgctgttcggacaaccgttaatcatgctgccaatcagtagcgatcagggcccgaatgcccg tcttatggagggaagaaaagttggaatgcaggtgcctcgtaatgagtccgatggatcattcaca agagaggatgtcgctgccacagtacaagcagtagcgatggaggaggatgggagccgtgtttt tacggctaatgcaaagaccatgcaagaaatagttgctgactccgcgtgtcacgaaaggtgcat cgatggatttatccaacagctaaggtcctataaagaa | 2 |
| TaUGTm_Protei n | MDDGSSSSSSPLRVVICPWLAFGHLLPCLDIAERLASRGHRVSFVS TPRNIARLPPVRPAVAPLVDYVALPLPRVDGLPEGAESTNDVPHDKF ELLRKAFDGLAAPFSEFLHAACAEGTGKRPDWLIVDSFHHWAAAAA VENKVPCVMLLLGAANVIATWARGVSEHAAAAVGKERSAAEAPSFE TERRKLMITQNASGMSLAERYFLTLMRSNLVAIRSCAEWEPESVAAL TTLAGKPVVTLGLLPPSPEGGRGISKQDAAVRWLDAQRDKSVVYVA LGSEVPLRVEQVHELALGLELSGASFLWALRKPPGMPDAAVLPPGF EERTRGRGLVVTGWVPQISVLAHGAVAAFLTHCGWNSTIEGLLFGQ PLIMLPISSDQGPNARLMEGRKVGMQVPRNESDGSFTREDVAATV QAVAMEEDGSRVFTANAKTMQEIVADSACHERCIDGFIQQLRSYKE | 3 |

(continued)

| Name | sequence (5' -> 3') | SEQ ID NO |
|------|---------------------|-----------|
| TaUGTm_DNA | ATGGACGACGGGTCTTCTAGCTCTAGCAGTCCGCTGCGTGTTGT TATTTGCCCGTGGCTGGCTTTTGGACACCTGCTTCCATGCTTAGA CATTGCAGAAAGATTAGCCAGCCGTGGACACAGAGTATCCTTCG TAAGTACGCCGCGTAATATAGCACGTTTACCCCCGGTCAGACCG GCAGTTGCGCCGCTGGTCGATTACGTAGCGCTACCTCTGCCCAG AGTGGACGGACTTCCGGAAGGGGCTGAATCTACAAACGACGTG CCCCATGATAAGTTCGAGCTTTTACGTAAGGCTTTCGACGGTTTG GCAGCGCCCTTCAGCGAATTTCTACACGCAGCGTGCGCGGAGG GCACAGGCAAAAGACCAGATTGGCTTATTGTGGATTCCTTCCAC CATTGGGCTGCGGCTGCGGCAGTGGAAAATAAAGTCCCGTGCG TCATGTTACTTTTAGGTGCAGCCAACGTGATCGCCACTTGGGCA CGTGGCGTGTCAGAGCATGCGGCCGCCGCGGTTGGAAAAGAAC GTAGTGCCGCAGAAGCTCCATCATTCGAAACCGAACGTCGTAAA CTGATGATAACTCAGAATGCATCAGGTATGTCATTAGCAGAGCGT TATTTCCTGACACTTATGAGATCAAACTTGGTTGCCATTCGTTCCT GTGCAGAATGGGAGCCTGAATCTGTGGCGGCACTAACCACTCTG GCCGGGAAGCCCGTAGTGACCTTAGGTTTGCTTCCACCGTCTCC TGAGGGAGGAAGGGGTATTTCCAAGCAAGACGCTGCCGTTAGG TGGCTGGACGCTCAGAGGGATAAGTCCGTTGTATACGTGGCGTT AGGGTCTGAAGTTCCCTTGAGAGTGGAGCAGGTACATGAGCTTG CGCTTGGACTAGAGTTGTCAGGTGCTAGCTTCCTGTGGGCGTTA CGTAAGCCACCCGGCATGCCTGACGCCGCAGTCTTGCCGCCGG GTTTTGAAGAGAGAACCAGGGGACGTGGTCTGGTTGTGACTGG CTGGGTACCACAAATCAGCGTCTTAGCACATGGCGCCGTTGCCG CTTTTCTGACACATTGCGGCTGGAATAGTACAATCGAAGGCTTGC TGTTCGGACAACCGTTAATCATGCTGCCAATCAGTAGCGATCAGG GCCCGAATGCCCGTCTTATGGAGGGAAGAAAAGTTGGAATGCAG GTGCCTCGTAATGAGTCCGATGGATCATTCACAAGAGAGGATGT CGCTGCCACAGTACAAGCAGTAGCGATGGAGGAGGATGGGAGC CGTGTTTTTACGGCTAATGCAAAGACCATGCAAGAAATAGTTGCT GACTCCGCGTGTCACGAAAGGTGCATCGATGGATTTATCCAACA GCTAAGGTCCTATAAAGAA | 30 |

(continued)

| Name | sequence (5' -> 3') | SEQ ID NO |
|---|---|---|
| Sucrose_syntha se_ Protein derived from Arabidopsis thaliana | MANAERMITRVHSQRERLNETLVSERNEVLALLSRVEAKGKGILQQ NQIIAEFEALPEQTRKKLEGGPFFDLLKSTQEAIVLPPVVVALAVRPR PGVWEYLRVNLHALVVEELQPAEFLHFKEELVDGVKNGNFTLELDF EPFNASIPRPTLHKYIGNGVDFLNRHLSAKLFHDKESLLPLLKFLRLH SHQGKNLMLSEKIQNLNTLQHTLRKAEEYLAELKSETLYEEFEAKFE EIGLERGWGDNAERVLDMIRLLLDLLEAPDPCTLETFLGRVPMVFN VVILSPHGYFAQDNVLGYPDTGGQVVYILDQVRALEIEMLQRIKQQ GLNIKPRILILTRLLPDAVGTTCGERLERVYDSEYCDILRVPFRTEKGI VRKWISRFEVVWPYLETYTEDAAVELSKELNGKPDLIIGNYSDGNLVA SLLAHKLGVTQCTIAHALEKTKYPDSDIYWKKLDDKYHFSCQFTADI FAMNHTDFIITSTFQEIAGSKETVGQYESHTAFTLPGLYRVVHGIDVF DPKFNIVSPGADMSIYFPYTEEKRRLTKFHSEIEELLYSDVENKEHLC VLKDKKKPILFTMARLDRVKNLSGLVEWYGKNTRLRELANLVVVGG DRRKESKDNEEKAEMKKMYDLIEEYKLNGQFRWISSQMDRVRNGE LYRYICDTKGAFVQPALYEAFGLTVVEAMTCGLPTFATCKGGPAEIIV HGKSGFHIDPYHGDQAADTLADFFTKCKEDPSHWDEISKGGLQRIE EKYTWQIYSQRLLTLTGVYGFWKHVSNLDRLEARRYLEMFYALKYR PLAQAVPLAQDD | 4 |
| Sucrose_syntha se_ nucleotide sequence derived from Arabidopsis thaliana | atggctaacgccgaacgtatgatcacgagggtacactcccaacgtgaacgtttaaatgaaact ttggtcagcgagcgtaacgaggttctagcactactttccagggtcgaagctaaggggaagggg atacttcagcaaaatcagataatcgcggagttcgaggctttaccggagcagaccagaaaaaa gttagagggtggcccgttctttgatctacttaaatctacacaggaggctattgtgctgccaccttgg gtcgccctggcagtcaggcctaggccaggcgtatgggagtaccttcgtgttaatttgcacgcatt agtagtggaggaattacagcctgccgaatttctacactttaaagaggagttagtggacggtgtc aagaatggaaacttcacccttgagcttgatttcgaaccattcaatgccagcatacccc gtcctac attacacaaatatattgggaacggggtcgactttctgaataggcatctatcagctaagttgttccat gacaaggaatcccttcttcctctattgaaatttttgagacttcacagtcatcaggggaagaacttg atgttaagcgagaaaatacaaaacttgaatacgttacaacatacgcttagaaaggcagagga gtatctggcggaactaaagagtgagactttgtacgaagagttcgaagccaagtttgaggaaat aggtcttgaacgtgggtggggcgacaatgctgaacgtgtcctggatatgatacgtcttttactgga cttattagaggcaccggacccatgtaccctagaaacattcttagggagggtacccatggttttca acgttgtcattcttagtccgcatggatattttgcccaagataacgtgttaggatacccc gacaccg ggggacaagttgtttatatttagatcaagtaagagcattagagattgagatgttacaaagaatta aacagcagggtctgaacataaaaccaaggatcttaattttgactcgtcttcttcctgatgcagtgg ggacgacgtgcggagagaggctggaacgtgtctacgattcagaatattgtgatatcctgagag tgccctttagaacagaaaaaggaatcgtgcgtaagtggatcagtagatttgaagtctggccata tctggagacatacaccgaagatgcagcggtcgaacttagcaaagagctaaacgggaaacc cgacctgataatcgggaactacagcgacgggaatttagttgccagtttacttgcccataagctg ggagtgacacagtgcacgatagcccacgcccttgaaaagacgaagtatcccgattctgacat atattggaaaaaacttgatgacaaataccattttttcatgccaattcactgctgatatctttgcaatga | 5 |

(continued)

| Name | sequence (5' -> 3') | SEQ ID NO |
|------|---------------------|-----------|
|  | atcacacggatttcatcattacttccacatttcaagagatcgcaggcagcaaagagacagttgg acaatatgagagtcatactgcattcacactacctggtctttatagagtcgtgcacggtatagacgt gtttgatcctaagttcaacatagtctcaccgggtgcggatatgtccatctactttccctacactgag gaaaagaggcgtttaaccaagtttcattcagagatagaagagttattatatagtgatgtggaaa ataaagagcatctatgcgtgttaaaagacaagaagaaacctatcctattcacaatggccagac ttgacagagtgaagaatctatccggcttggttgaatggtacggcaagaacacgcgtcttcgtga gctagcgaaccttgtcgtagtaggaggtgacagaagaaaggaatccaaagacaacgaaga gaaagcggagatgaaaaagatgtacgatctaatcgaagaatacaaattaaatggtcagtcc gttggatctcctctcagatggatagagtccgtaatggagaactatacagatatatatgcgacacc aaaggcgcgtttgtgcagccggcattgtacgaggcctttggtctgactgtcgtcgaggccatgac ctgcggccttcctacattcgcgacctgcaaaggtggtccagctgagattattgtccatgggaaat ctggattccacatagaccatatcatggtgaccaggccgctgatacacttgcagatttttttactaa gtgtaaggaggacccttcacattgggatgaaatatccaaaggggggactgcagcgtatcgagg agaaatatacctggcaaatatatagccaaaggctgttgaccctaacgggcgtttacgggttctg gaagcacgtttccaatctagataggctagaggcgaggcgttacctggaaatgttttacgcgttga aatacaggcctctagcacaagcagtaccactggcgcaggacgat |  |
| SrUGT76G1_Pr otein | MENKTETTVRRRRRIILFPVPFQGHINPILQLANVLYSKGFSITIFHTN FNKPKTSNYPHFTFRFILDNDPQDERISNLPTHGPLAGMRIPIINEHG ADELRRELELLMLASEEDEEVSCLITDALWYFAQSVADSLNLRRLVL MTSSLFNFHAHVSLPQFDELGYLDPDDKTRLEEQASGFPMLKVKDI KSAYSNWQILKEILGKMIKQTKASSGVIWNSFKELEESELETVIREIP APSFLIPLPKHLTASSSSLLDHDRTVFQWLDQQPPSSVLYVSFGSTS EVDEKDFLEIARGLVDSKQSFLWVVRPGFVKGSTWVEPLPDGFLG ERGRIVKWVPQQEVLAHGAIGAFWTHSGWNSTLESVCEGVPMIFS DFGLDQPLNARYMSDVLKVGVYLENGWERGEIANAIRRVMVDEEG EYIRQNARVLKQKADVSLMKGGSSYESLESLVSYISSL | 31 |

(continued)

| Name | sequence (5' -> 3') | SEQ ID NO |
|---|---|---|
| SrUGT76G1_D NA | ATGGAAAACAAGACGGAAACCACTGTTAGAAGgAGAAGAAGgATT ATcTTGTTCCCAGTTCCATTCCAAGGTCATATTAATCCAATTTTGCA ATTGGCTAATGTcTTGTATTCTAAAGGTTTCTCTATTACTATaTTCCA TACTAATTTCAATAAACCAAAgACTTCTAATTATCCACATTTCACTTT CAGATTCATaTTGGATAATGATCCACAAGATGAAAGAATTTCTAATT TGCCAACTCATGGTCCATTGGCTGGTATGAGAATTCCAATTATTAA TGAACATGGTGCTGATGAATTGAGAAGAGAATTGGAATTGTTGAT GTTGGCTTCTGAAGAAGATGAgGAAGTTTCTTGTTTGATTACTGAT GCTTTGTGGTATTTCGCTCAATCTGTTGCTGATTCTTTGAATTTGA GAAGATTGGTTTTGATGACaTCTTCTTTGTTCAATTTCCATGCTCA TGTTTCTTTaCCACAATTCGATGAATTGGGTTATTTaGATCCAGATG ATAAgACTAGATTGGAAGAACAAGCTTCTGGTTTCCCAATGTTGA AAGTTAAAGATATTAAATCTGCTTATTCTAATTGGCAAATcTTGAAA GAAATcTTGGGTAAgATGATTAAACAAACTAAAGCTTCTTCaGGTG TTATTTGGAATTCTTTCAAAGAATTGGAAGAgTCTGAATTGGAAAC TGTTATTAGAGAAATTCCAGCTCCATCTTTCTTGATTCCATTGCCA AAACATTTGACTGCTTCaTCTTCcTCTTTGTTaGATCATGATAGAAC aGTTTTCCAATGGTTGGATCAgCAACCACCATCTTCTGTcTTGTAT GTTTCTTTCGGTTCTACTTCTGAAGTTGATGAgAAAGATTTCTTGG AAATTGCTAGAGGTTTGGTTGATTCTAAACAATCTTTCTTaTGGGT TGTTAGACCAGGTTTCGTTAAAGGTTCTACaTGGGTTGAACCATT GCCAGATGGTTTCTTGGGTGAAAGAGGTAGAATTGTTAAATGGGT TCCACAgCAAGAAGTTTTGGCTCATGGTGCTATTGGTGCTTTCTG GACTCATTCTGGTTGGAATTCTACTTTGGAATCTGTTTGTGAAGG TGTTCCAATGATTTTCTCTGATTTCGGTTTGGATCAACCATTGAAT GCTAGATATGTCTGATGTcTTGAAAGTTGGTGTTTATTTGGAAAA TGGTTGGGAAAGAGGTGAAATTGCTAATGCTATTAGAAGAGTTAT GGTTGATGAgGAAGGTGAATATATTAGACAAAATGCTAGAGTTTTG AAACAgAAAGCTGATGTTTCTTTGATGAAAGGTGGTTCTTCTTATG AATCTTTGGAATCTTTGGTATCCTACATCTCTTCCTTG | 32 |

(continued)

| Name | sequence (5' -> 3') | SEQ ID NO |
|---|---|---|
| Sucrose_syntha se_ Protein derived from Glycine max | MATDRLTRVHELRERLDETLTANRNEILALLSRIEAKGKGILQHHQVI AEFEEIPEENRQKLTDGAFGEVLRSTQEAIVLPPWWALAVRPRPGV WEYLRVNVHALVVEELQPAEYLHFKEELVDGSSNGNFVLELDFEPF NAAFPRPTLNKSIGNGVQFLNRHLLAKLFHDKESLHPLLEFLRLHSV KGKTLMLNDRIQNPDALQHVLRKAEEYLGTVPPETPYSEFEHKFQE IGLERGWGDNAERVLESIQLLLDLLEAPDPCTLETFLGRIPMVFNVVI LSPHGYFAQDNVLGYPDTGGQVVYILDQVRALENEMLHRIKQQGL DIVPRILIITRLLPDAVGTTCGQRLEKVFGTEHSHILRVPFRTEKGIVR KWISRFEVWPYLETYTEDVAHELAKELQGKPDLIVGNYSDGNIVASL LAHKLGVTQCTIAHALEKTKYPESDIYWKKLEERYHFSCQFTADLFA MNHTDFIITSTFQEIAGSKDTVGQYESHTAFTLPGLYRVVHGIDVFD PKFNIVSPGADQTIYFPHTETSRRLTSFHPEIEELLYSSVENEEHICV LKDRSKPIIFTMARLDRVKNITGLVEWYGKNAKLRELVNLVVVAGDR RKESKDLEEKAEMKKMYGLIETYKLNGQFRWISSQMNRVRNGELY RVICDTRGAFVQPAVYEAFGLTVVEAMTCGLPTFATCNGGPAEIIVH GKSGFHIDPYHGDRAADLLVDFFEKCKLDPTHWDKISKAGLQRIEE KYTWQIYSQRLLTLTGVYGFWKHVSNLDRRESRRYLEMFYALKYRK LAESVPLAAE | 33 |
| Sucrose_syntha se_ DNA drived from Glycine max | ATGGCCACGGACAGACTGACTCGTGTACACgaaTTACGTGAGAG GTTGGACGAGACGCTAACAGCAAACAGGAACGAGATACTGGCAT TGCTTTCAAGGATAGAAGCAAAGGGTAAGGGAATTCTACAGCATC ACCAAGTCATAGCAGAATTCGAAGAAATACCTGAGGAGAATCGTC AAAAGTTAACAGATGGGGCCTTCGGAGAAGTCCTAAGGAGTACA CAAGAGGCAATAGTTCTACCTCCGTGGGTGGCACTAGCTGTTAG ACCTAGGCCTGGAGTCTGGGAATATCTAAGGGTGAACGTTCATG CTCTGGTTGTAGAGGAACTACAGCCAGCAGAATATCTTCACTTCA AGGAAGAACTTGTTGACGGCTCATCAAATGGCAATTTCGTGTTG GAACTGGATTTTGAGCCGTTTAACGCCGCTTTCCCGAGGCCGAC GCTAAATAAAAGTATAGGTAATGGTGTTCAATTTCTTAACAGGCAT CTTctaGCCAAATTGTTCCACGATAAAGAGAGTCTTCACCCCTTATT GGAATTTCTACGTCTACACTCTGTTAAGGGCAAGACATTGATGTT AAATGATAGGATACAGAATCCAGACGCACTTCAGCATGTTCTGAG AAAGGCCGAGGAATACCTAGGAACCGTACCCCCGGAAACTCCTT ACTCAGAATTCGAACATAAGTTTCAAGAGATCGGTCTAGAAAGAG GATGGGGTGATAACGCTGAACGTGTATTAGAGTCTATACAGCTTT TGCTTGACTTACTAGAAGCGCCTGACCCCTGCACTCTAGAAACAT TCTTGGGAAGGATTCCAATGGTCTTCAATGTCGTAATACTTAGTC CACACGGGTATTTTGCTCAAGATAACGTACTTGGATATCCTGACA CTGGGGGCCAAGTAGTATACATTCTAGACCAAGTCAGGGCTTTG GAGAATGAGATGCTACACAGAATTAAGCAACAAGGTCTTGACATA GTACCTCGTATATTGATAATTACTAGATTGCTACCGGATGCCGTTG GGACGACGTGTGGGCAACGTCTAGAGAAAGTGTTCGGGACTGA ACACAGCCATATCTTGCGTGTACCGTTCAGGACCGAAAAAGGGA TTGTGCGTAAATGGATTAGTAGATTTGAGGTTTGGCCGTACCTAG | 34 |

(continued)

| Name | sequence (5' -> 3') | SEQ ID NO |
|---|---|---|
| | AAACGTATACGGAAGATGTCGCGCATGAGTTGGCTAAGGAGCTG CAAGGTAAACCCGACTTGATCGTCGGTAACTATTCCGACGGTAAC ATTGTTGCGTCCCTTCTTGCACATAAACTAGGCGTTACACAGTGT ACTATCGCTCATGCCTTGGAAAAAACGAAATATCCCGAATCTGAC ATTTACTGGAAGAAATTGGAAGAGAGATATCATTTCTCATGTCAAT TCACAGCAGACCTTTTCGCTATGAACCACACGGACTTCATTATAA CTTCTACATTTCAAGAGATAGCGGGCTCAAAGGATACTGTTGGCC AGTATGAAAGTCATACGGCTTTTACATTGCCGGGCCTGTATAGGG TCGTACATGGCATTGACGTTTTCGACCCTAAATTCAACATAGTTTC CCCAGGTGCGGACCAGACCATTTACTTCCCACACACTGAAACCT CAAGGCGTTTGACGTCTTTCCATCCAGAGATAGAAGAATTACTGT ATTCAAGTGTTGAGAACGAAGAGCATATATGCGTACTTAAGGATC GTTCTAAACCAATTATCTTTACGATGGCGAGGCTTGATAGAGTCA AGAATATCACGGGCCTTGTTGAGTGGTACGGGAAGAATGCCAAG CTAAGAGAGCTGGTGAATCTTGTCGTTGTCGCTGGAGACAGAAG AAAGGAAAGTAAGGACCTTGAGGAGAAAGCAGAGATGAAGAAAA TGTATGGCTTAATCGAAACATATAAACTAAATGGTCAGTTCAGGTG GATATCAAGTCAGATGAACCGTGTACGTAATGGCGAATTATACAG AGTCATCTGTGACACTAGGGGTGCTTTCGTACAGCCGGCTGTAT ACGAGGCTTTTGGCCTGACAGTAGTGGAAGCCATGACGTGCGG CCTTCCTACGTTCGCAACGTGTAACGGGGGACCAGCTGAAATCA TTGTTCACGGCAAAAGCGGATTCCACATAGACCCCTACCACGGC GATAGAGCCGCCGACTTGTTAGTGGATTTCTTCGAGAAATGTAAA CTAGACCCTACCCACTGGGACAAAATCAGTAAAGCAGGATTACAA CGTATTGAAGAAAATATACATGGCAAATCTACTCACAACGTTTGT TAACTTTAACCGGTGTGTATGGTTTTTGGAAGCACGTGAGCAACC TTGACAGGAGAGAAAGCAGGCGTTACCTGGAAATGTTcTACGCcT TGAAATATAGGAAATTGGCTGAGTCAGTCCCTCTGGCTGCCGAG | |

In the present disclosure, the biocatalyst containing the enzyme may include at least one selected from the group consisting of the enzyme protein, the cells of the microorganism, a culture of the microorganism, a lysate of the microorganism, and an extract of the lysate or the culture.

The biocatalyst relates to a recombinant microorganism containing a first UDP-glycosyl transferase, specifically a recombinant microorganism which further expresses one or more enzymes selected from the group consisting of UDP-glycosyltransferase 76G1 (SrUGT76G1) and sucrose synthase, cells of the microorganisms, cultures of the microorganisms, lysates of the microorganisms, and extracts of the lysates or the cultures. In addition, the biocatalyst may be a transformed microorganism that expresses all of TaUGT orTaUGTm, SrUGT76G1 and sucrose synthase, and has an activity of converting to Rebaudioside M from stevia, cells of the microorganism, cultures of the microorganism, lysates of the microorganism, and extracts of the lysates or the cultures.

The lysate refers to the lysate obtained by crushing the cells of the microorganism producing UDP-glucosyl transferase or the supernatant obtained by centrifuging the lysate, and includes enzymes produced from the microorganism producing the UDP-glucosyl transferase.

The culture contains an enzyme produced from a microorganism that produces the UDP-glucosyltransferase, and may contain the microbial cells or cell-free form not containing the microbial cells. Unless otherwise specified in the present specification, it means that the used microorganisms producing UDP-glucosyltransferase include cells of the microorganism, cultures of the microorganism, lysates of the microorganism, supernatant of the lysate, and extracts thereof.

The biocatalyst according to the present invention may be a microbial cell or a dried cell thereof with enzyme activity. The dried microbial cells according to the present invention have low moisture content, and high storage stability and conversion activity. The dried cells have a decreased moisture content of the same weight of cells, making them easy to store and distribute, and maintain conversion activity stably for a long period of time, thereby making them particularly suitable for mass production.

The dried yeast cell powder according to the present invention can stably maintain the conversion activity of recombinant yeast in a dry state for a long period of time. In other words, even if stored at room temperature, it can be stored and maintained without excessively lowering the enzyme titer at drying.

In addition, the production of steviol glycosides according to the present invention can be carried out through a whole-cell reaction using microbial cells or dried cells themselves having an converting activity of steviol glycosides, especially Rebaudioside M. The biocatalyst not only has an conversion activity of steviol glycosides, especially Rebaudioside M, but can also produce steviol glycosides, especially Rebaudioside M, at a high conversion rate in a whole-cell conversion reaction.

When the microorganism having an conversion activity of steviol glycosides, especially Rebaudioside M in the present invention is yeast, it exhibits a low conversion activity in the whole-cell reaction due to its thick cell wall, so the reaction using dried cells compared to the cells separated from microbial culture has a high conversion rate. Moreover, the whole-cell conversion reaction can be provided with a liquid raw material containing the substrate to be converted, and can be performed by mixing the liquid raw material with separated cells or dried cells. Thus, the cell wall permeability to the liquid substrate can be increased by treating the cells, or adding substances that can increase the cell wall permeability of the recombinant yeast cells. An example of a method of treating microbial cells to have a high permeability of cell wall to a liquid substrate is to perform a drying treatment. The method of the present invention does not have the purpose of increasing and maintaining the viability of such microorganisms, but rather, in is preferable to kill the microorganisms themselves and preserve only the enzyme activity possessed by of the microorganisms.

In an example of the present invention, the drying method of the microbial cells is not particularly limited, and for example, may include freeze drying, hot air drying using air or wind, spray drying, etc. but is not limited thereto. As for a drying method of the microbial cell, the drying treatment with a high permeability to liquid substrate is more preferable, and for example, the freeze-drying may be used. In the case of hot air drying or spray drying, the inlet and outlet temperatures of the dryer can be performed within a range that does not significantly reduce enzyme activity.

The freeze-drying of the microbial cells can be performed using a conventional freeze-drying method, but since it is important to maintain the conversion activity of the enzyme, the temperature range is -90 to 40°C, or -90°C to -5°C, or preferably -60°C to - 40°C, -50 to 40°C, -60 to 20°C, and -50 to 20°C, and the drying can be preferable performed at a temperature range of -60°C to 40°C under a pressure of less than 10 mtorr. Additionally, cryoprotectant, etc. may not be added in the freeze-drying process of microbial cells. The dried beads were prepared by completely removing moisture from the frozen beads in a freeze dryer.

The dried powder of microbial cells obtained in the present invention has the advantage of maintaining an conversion activity of the microorganism immediately after drying for more than one year, or maintaining at least 90% of it, even if stored at room temperature (25°C). That is, based on 100% of the conversion activity of the cells immediately after drying, or based on 100% of the conversion activity of the cells to steviol glycosides at the start of storage, dried powder of microbial cells have 90% or more, 95% or more, 97% or more, or 98% or more of the conversion activity, when stored at room temperature for 9 weeks. Accordingly, the freeze-dried cells according to the present invention can maintain a high conversion activity for a long period of time. In contrast, the enzyme activity of cell lysates or crude enzyme solution obtained from them is almost lost 24 hours after cell disruption, and the residual activity decreases to less than 10%.

Recombinant microorganisms described herein include fungal cells or bacterial cells. In some embodiments, the bacterial cell comprises a microorganism of the genus *Escherichia, Lactobacillus, Lactococcus, Cornebacterium, Acetobacter, Acinetobacter, Aspergillus, or Pseudomonas. The fungal cells are yeast cells, for example, Saccharomyces genus, Schizosaccharomyces genus, Yarrowia genus, Candida genus, Ashbya genus, Cyberlindnera genus, Pichia genus, Kluyveromyces genus, and Hansenula genus.* Specifically, the microorganisms include *E. coli,* Saccharomyces genus strains, (e.g., *Saccharomyces cerevisiae*) or Pichia genus strains (e.g., *Pichia pastoris*), etc., but is not limited to.

For the expression of TaUGTm, SrUGT76G1 and/or sucrose synthase, the recombinant microorganism provided by the present invention may be one in which exogenous TaUGTm, SrUGT76G1 and/or sucrose synthase genes have been transformed. The genes of TaUGT or TaUGTm, SrUGT76G1 and/or sucrose synthase may be cloned into a vector and transformed into a microorganism, but are not limited thereto.

As used herein, the term "transformation" refers to introducing a vector containing a nucleic acid molecule encoding a target protein into a host cell so that the protein encoded by the nucleic acid molecule can be expressed in the host cell. As long as the transformed nucleic acid molecule can be expressed in the host cell, it can include both of these molecules, regardless of whether they are inserted into the chromosome of the host cell or located outside the chromosome. Additionally, the nucleic acid molecule includes DNA and RNA that encode the target protein. The nucleic acid molecule may be introduced in any form, as long as it can be introduced into a host cell and expressed. For example, the nucleic acid molecule can be introduced into the host cell in the form of an expression cassette, which is a genetic structure containing all elements necessary for self-expression. The expression cassette may typically include a promoter, a transcription termination signal, a ribosome binding site, and a translation termination signal that are operably linked to the nucleic acid molecule. The expression cassette may be in the form of an expression vector capable of self-replication. In addition, the nucleic acid molecule may be introduced into the host cell in its own form and operably linked to the nucleic acid sequence required for expression in the host cell, but is not limited thereto.

The vector used in this application is not particularly limited, and any vector known in the art can be used. Examples of commonly used vectors include plasmids, cosmids, viruses, and bacteriophages in a natural or recombinant state. A polynucleotide encoding a target polypeptide can be inserted into a chromosome through a vector for intracellular chromosome insertion. Insertion of the polynucleotide into the chromosome may be accomplished by any method known in the art, for example, homologous recombination, but is not limited thereto. A selection marker may be additionally included to confirm whether the chromosome has been inserted.

In addition, the term "operably linked" as used herein means that the gene sequence is functionally linked to a promoter sequence that initiates and mediates transcription of the nucleic acid molecule encoding the target protein of the present invention.

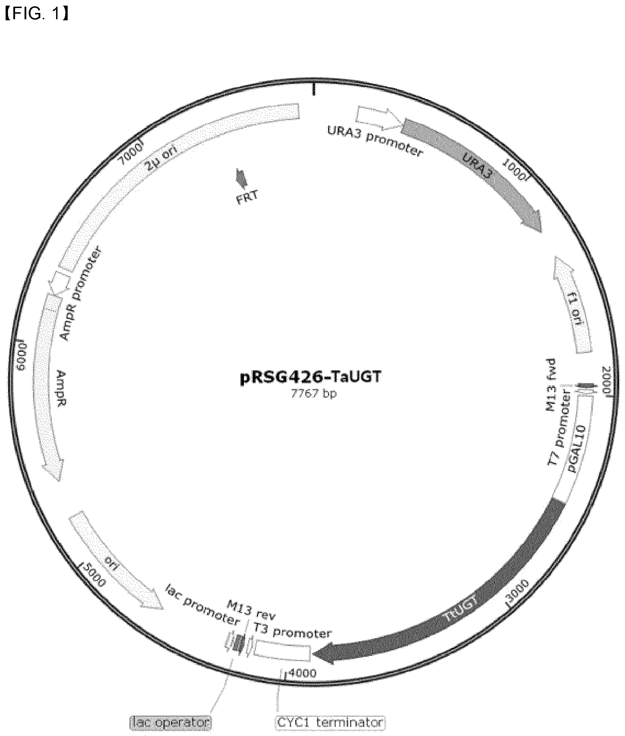

An example of a recombinant vector containing a nucleic acid molecule encoding a glycosyltransferase protein that transfers glucose to steviol glycosides according to an example of the present invention is shown in the cleavage map of FIG. 1. For example, it may include a nucleic acid molecule encoding the enzyme according to the present invention, and a GAL10 promoter and CYC1 terminator as a transcriptional regulatory sequence that can be operably linked to the nucleic acid molecule. The transcriptional promoter may include GAL10, GAL1, GAL2, TEF1, GPD1, TDH3 promoters and the like, and the transcriptional terminator may include CYC1, TEF1, PGK1, and the like.

The method of transforming the vector of the present invention includes any method of introducing a nucleic acid molecule into a cell, and can be performed by selecting an appropriate standard technique as known in the art depending on the host cell. For example, electroporation, calcium phosphate ($CaPO_4$) precipitation, calcium chloride ($CaCl_2$) precipitation, microinjection, polyethylene glycol (PEG) method, DEAE-dextran method, cationic liposome method, and lithium acetate. -DMSO method, etc. can be used, but not limited to.

The composition for producing steviol glycosides or the method for producing steviol glycosides of the present invention performs a conversion reaction at a high substrate concentration, thereby providing high conversion rate of Rebaudioside M and being applicable to industrial mass production.

In this specification, the reaction substrate (raw reaction material) used in the conversion reaction for producing steviol glycosides may include one or more Steviol glycoside substrates selected from the group consisting of stevioside and Rebaudioside A, for example, stevioside or Reb A as a single substrate, or a mixture of these substrates. The mixed substrate may be a mixture of stevioside and Reb A, or a stevia extract containing stevioside or Reb A.

The term,"Stevia" in the present specification may refer to a stevia extract, and specifically, Rebaudioside A may be included in an amount of 30 (w/w)% or more, 35 (w/w)% or more, 40 (w/w)% or more, 50 (w/w)% or more, 55 (w/w)% or more, 58 (w/w)% or more, 60 (w/w )% or more, 70(w/w)% or more, or 80(w/w)% or more, or specifically 50 to 99.5 (w/w)%, 50 to 99 (w/w)%, 50 to 95 (w/w)%, 50 to 90 (w/w)%, 50 to 85 (w/w) %, 50 to 80 (w/w)%, 50 to 70 (w/w)%, 50 to 65 (w/w)%, 50 to 62 (w/w)%, 55 to 99.5 (w/w) %, 55 to 99 (w/w)%, 55 to 95 (w/w)%, 55 to 90 (w/w)%, 55 to 85 (w/w)%, 55 to 80 (w/w) %, 55 to 70 (w/w)%, 55 to 65 (w/w)%, 55 to 62 (w/w)%, 58 to 99.5 (w/w)%, 58 to 99 (w/w) %, 58 to 95 (w/w)%, 58 to 90 (w/w)%, 58 to 85 (w/w)%, 58 to 80 (w/w)%, 58 to 70 (w/w) %, 58 to 65 (w/w)%, 58 to 62 (w/w)%, such as 60 (w/w)%, based on the total mass of steviol glycosides contained in the stevia extract, and stevioside may be included in the stevia extract in a remaining content with excluding the Rebaudioside A content, based on the total volume of the extract, and may specifically be included in a content of 30 to 50 (w/w)%, 30 to 45 (w/w)%, 30 to 42 (w/w)%, 35 to 50 (w/w)%, 35 to 45 (w/w)%, 35 to 42 (w/w)%, 38 to 50 (w/w)%, 38 to 45 (w/w)% or 38 to 42 (w/w)%, such as 40 (w/w)%, based on 100% by weight of steviol glycoside, but is not limited thereto.

The present invention can produce Reb M with high purity and high conversion rate and productivity in an industrial mass production process using a large amount of reaction substrate. In this specification, the reaction substrate used in the conversion reaction to produce steviol glycosides may be provided in liquid form at 15 g/L or more, 20 g/L or more, 25 g/L or more, 50 g/L or more, 75 g/L or more, or 100 g/L or more, for examples 15 to 200 g/L, 20 to 200 g/L, 30 to 200 g/L, 40 to 200 g/L, 50 to 200 g/L, 60 to 200 g/L, 70 to 200 g/L, 80 to 200 g/L, 90 to 200 g/L, 15 to 150 g/L, 20 to 150 g/L, 30 to 150 g/L, 40 to 150 g/L, 50 to 150 g/L, 60 to 150g/L, 70 to 150 g/L, 80 to 150g/L, or 90 to 150 g/L.

The reaction substrate may further include reaction raw materials including one or more selected from the group consisting of stevia extract, uridine diphosphate glucose (UDP), and sucrose. In one embodiment, the conversion of Reb A to Reb D in the composition for producing steviol glycosides may further include $MgCl_2$ and/or $MnCl_2$.

The stevia extract of the present invention may contain steviol glycosides in an amount of 80 to 99 (w/w)%, 80 to 97 (w/w)%, 80 to 96 (w/w)%, 80 to 95 (w/w)%, 85 to 99 (w/w)%, 85 to 97 (w/w)%, 85 to 96 (w/w)%, 85 to 95 (w/w)%, 90 to 99 (w/w)%, 90 to 97 (w/w)%, 90 to 96 (w/w)%, 90 to 95 (w/w)%, 93 to 99 (w/w)%, 93 to 97 (w/w)%, 93 to 96 (w/w)%, 93 to 95 (w/w)%, 94 to 99 (w/w)%, 94 to 97 (w/w)%, 94 to 96 (w/w)%, 94 to 95 (w/w)%, 95 to 99 (w/w)%, 95 to 97 (w/w)%, or 95 to 96 (w/v)%, such as 95 (w/wv)%, but is not limited thereto. The steviol glycosides may include Rebaudioside A and stevioside.

The reaction raw material used in the composition for producing Rebaudioside M provided by the present invention and/or the reaction raw material used in the method for producing Rebaudioside M provided by the present invention may be a reaction raw material solution, and sucrose is used at a concentration of 500 to 2000 mM, 500 to 1500 mM, 500 to 1200 mM, 500 to 1100 mM, 500 to 1050 mM, 500 to 1000 mM, 600 to 2000 mM, 600 to 1500 mM, 600 to 1200 mM, 600 to 1100 mM, 600 to 1050 mM, or 600 to 1000 mM, based on the reaction raw material, but is not limited thereto.

The composition may further include reaction raw materials including one or more selected from the group consisting of stevia extract, uridine diphosphate glucose (UDP), and sucrose. In one embodiment, for the conversion reaction of Reb A to Reb D, the composition for producing steviol glycosides may further include $MgCl_2$ and/or $MnCl_2$.

In steviol glycosides containing Rebaudioside M, which is the reaction product in the present invention, the purity of Rebaudioside M is 50% by weight or more, 55% by weight or more, 60% by weight or more, 65% by weight or more, 70% by weight or more, 75% by weight or more, 80% by weight or more or 85% by weight or more, or specifically, 50% to 99% by weight, 55% to 99% by weight, 60% to 99% by weight, 65% to 99% by weight, 70% to 99% by weight, 75% to 99% by weight, 80% by weight % to 99% by weight, 85% to 99% by weight, or 87 to 99% by weight.

The conversion rate of Rebaudioside M produced and/or manufactured by the composition for producing Rebaudioside M provided by the present invention and/or the Rebaudioside M production method provided by the present invention may be 110% to 200%, 115% to 200%, 120% to 200%, 125% to 200%, 130% to 200% or 135% to 200%, such as 125%, 129%, 135% or 136%, based on the weight of the stevia extract contained in the reaction raw material, but not limited thereto.

The content of Rebaudioside M in the composition for producing Rebaudioside M provided by the present invention and/or the steviol glycosides produced by the method for producing Rebaudioside M provided by the present invention may be 80 to 99% by weight, 85 to 99% by weight, 87 to 99% by weight, 90 to 99% by weight, 94 to 99% by weight or 95 to 99% by weight, for example, 87.7% by weight, 90.2% by weight, 94.8% by weight or 95.4% by weight.

The conversion reaction according to the present invention can be carried out in an aqueous system at a temperature of 20 to 60°C and pH of 4 to 9. Preferably, the reaction may be performed in an aqueous system with a reaction temperature of 25 to 40°C and a pH of 6.0 to 8.0, more preferably in an aqueous system with a temperature of 30°C and pH 7.2. According to a preferred example of the invention, the reaction can be performed in a phosphate buffer solution at pH 7.2.

The pH of the stevia extract contained in the reaction raw material of the composition for producing Rebaudioside M provided by the present invention or the pH of the conversion reaction is pH 4 to 9, pH 4 to 8.5, pH 4 to 8.2, pH 4 to 8, pH 4 to 7.8, pH 4 to 7.5, pH 4 to 7, pH 4 to 6.7, pH 4 to 6.5, pH 4 to 6.3, pH 4 to 6.2, pH 5 to 7, pH 5 to 6.7, pH 5 to 6.5, pH 5 to 6.3, pH 5 to 6.2, pH 5.5 to 7, pH 5.5 to 6.7, pH 5.5 to 6.5, pH 5.5 to 6.3, pH 5.5 to 6.2, pH 5.7 to 7, pH 5.7 to 6.7, pH 5.7 to 6.5, pH 5.7 to 6.3, pH 5.7 to 6.2, pH 5.9 to 7, pH 5.9 to 6.7, pH 5.9 to 6.5, pH 5.9 to 6.3, pH 5.9 to 6.2, pH 6.0 to 7, pH 6.0 to 6.7, pH 6.0 to 6.5, 6.0 to 6.3 and pH 6.0 to 6.2, but is not limited thereto.

In the present specification, the reaction temperature in the composition for producing steviol glycosides or the method for producing steviol glycosides according to the present invention may be 20 to 60 °C, 20 to 50 °C, 20 to 45 °C, 20 to 40 °C, 20 to 37 °C, 20 to 35 °C.

[ADVANTAGEOUS EFFECTS]

The present invention relates to a transformed microorganism that expresses one or more enzymes selected from the group consisting of exogenous TaUGTm, SrUGT76G1 and sucrose synthase and has the activity of converting Rebaudioside M from stevia, and to a method for producing Rebaudioside M using the same, The transformed microorganism has a high productivity of Rebaudioside M.s

[BRIEF DESCRIPTION OF THE DRAWINGS]

Fig. 1 is a cleavage map of a vector for preparing a yeast strain producing TaUGTm enzyme according to an example of the present invention.
Fig. 2 shows a graph of the results obtained by analyzing the reaction product of TaUGTm enzyme according to an example of the present invention by HPLC analysis.

[MODE FOR INVENTION]

The present invention will be explained in more detail through the following examples, but it is not intended that the scope of the invention be limited to the following examples.

**Example 1. Production of microorganisms expressing wild-type enzyme**

1-1: Construction of vector expressing wild type enzyme

A gene encoding a wild-type enzyme protein having an amino acid sequence of SEQ ID NO: 1 derived from *Triticum aestivum* was synthesized (Gblock synthesis). The polynucleotide sequence of the synthesized gene is shown in SEQ ID NO: 2. The synthesized gene was subcloned into a plasmid expressed in *S. cerevisiae* to produce a form capable of gene expression.

Specifically, the vector prepared to test protein activity was pRS426 vector containing a Ura auxotrophic marker (selection marker) that could be selected in S. cerevisiae, the GAL10 promoter was used as the promoter, and CYC1 was used as the terminator.

The newly synthesized gene (IDT, gBlock) of SEQ ID NO: 2 was cloned into the plasmid. For cloning TaUGT gene, a pair of primers (SEQ ID NO: 19 and SEQ ID NO: 19) was synthesized to overlap the GAL10 promoter (SEQ ID NO: 16) and cyc1 terminator B (SEQ ID NO: 17), and the enzyme gene was amplified through PCR amplification. The genes were subcloned through Gibson assembly (HIFi DNA master mix, NEW ENGLAND BIOLABS), and the primer information used is shown in Table 2 below.

The gene in the form of a subcloned plasmid was selected and amplified by being transformed to into E. coli (DH5a), and the sequence of the amplified plasmid was analyzed to confirm that the gene expressing the enzyme was accurately subcloned.

[Table 2]

| Name | Nucleotide sequence (5'->3') | SEQ ID NO |
|---|---|---|
| Reverse primer of GAL10 promoter | catcaattcttactttttttttggatgg | 16 |
| Forward primer of CYC1 terminator | tgattgtcgatatcatgtaattagttatgtc | 17 |
| Forward primer of TaUGT connecting with GAL10 promoter | catccaaaaaaaaagtaagaattgatggacgacgggtcttctag | 18 |
| Reverse primer of TaUGT connecting with CYC1 terminator | ctaattacatgatatcgacaatcattctttataggaccttagctgttg | 19 |
| Forward primer of EUGT11 connecting with GAL10 promoter | catccaaaaaaaaagtaagaattgatggactccggctacagttc | 20 |
| Reverse primer of EUGT11 connecting with CYC1 terminator | ctaattacatgatatcgacaattagtccttataagaacgtagctg | 21 |
| Forward primer of HvUGT connecting with GAL10 promoter | catccaaaaaaaaagtaagaattgatggacggcgacggaaac | 22 |
| Reverse primer of HvUGT connecting with CYC1 terminator | ctaattacatgatatcgacaatcaagctttgtaggaacgcagttg | 23 |

The transformation to introduce the gene into S. cerevisiae CENPK2-1c (Euroscarf) was performed through heat shock by treating with 0.1M LiAc (Lithium Acetate). The transformed strain was selected on SC_Ura medium, and the selected colonies were cultured to confirm enzyme activity.

The prepared recombinant plasmid was transformed into S. cerevisiae CENPK2-1c (Euroscarf) strain. Recombinant strains with the inserted gene cassette were selected using a selection marker on SC-Ura solid medium (SC_Ura medium + 2% agar). The composition of SC-Ura solid medium included YNB 6.7 g/L, Drop out mix 0.7 g/L, Glucose 50 g/L, and agar 20g/L, and the pH was adjusted to 6.0.

1-2: Culture of microorganism

The yeast transformed with the plasmid prepared in Example 1-1 was selected in SC (synthetic complete) medium using URA-drop out mix, and was induced to express enzyme through liquid culture.

Specifically, colonies selected from SC_Ura solid medium were inoculated into a test tube containing 3 mL SC_Ura liquid medium and were cultured overnight. The concentration of the cultured cells was measured with $OD_{600}$ absorbance, and the cultured cells were inoculated in a 250 mL flask containing 25 mL of SC-Ura liquid medium until the OD600 absorbance was 0.1 (final), and primarily incubated for 24 hours (30°C, 240 rpm).

To induce enzyme expression in the primary cultured cells, the cells were cultured secondly for 24 hours with adding an equal amount of YPG medium. The secondary cultured cells were collected by centrifugation (4,000 rpm, 10 min). The composition of SC_Ura liquid medium included YNB (yeast nitrogene base) 6.7 g/L, Drop-out mix 0.7 g/L, Glucose 50 g/L, and MES 50 mM, and the medium pH was adjusted to 6.0 using NaOH. The YPG medium composition included 20 g/L of Bacto peptone, 10 g/L of Yeast Extract, 20 g/L of Galactose, and 100 mM Phosphate buffer (sodium salt), and the pH of the medium was adjusted to 6.0.

1-3: Production of microorganisms expressing EUGT11 and HvUGT

The EUGT11 gene (SEQ ID NO: 13) from *Oryza sativa,* which has known activity, and the HvUGT gene (SEQ ID NO: 15) from *Hordeum vulgare* were synthesized (Gblock synthesis), and the genes were amplified in the same manner as the TaUGT gene and cloned into the pRS426 vector. To induce enzyme expression under the same conditions, the GAL10 promoter was used as the promoter and CYC1 as the terminator.

Specifically, the forward primer of EUGT11 linked to the GAL10 promoter (SEQ ID NO: 20), the reverse primer of EUGT11 linked to the CYC1 terminator (SEQ ID NO: 21), the forward primer of HvUGT linked to the GAL10 promoter (SEQ ID NO: 22), and the reverse primer of HvUGT linked to the CYC1 terminator (SEQ ID NO: 23) were used. The used primer information is shown in Table 2 above, and Sc refers to *Saccharomyces cerevisiae.*

Thereafter, S. cerevisiae CENPK2-1c (Euroscarf) was transformed, and strain selection and microbial culture were performed using substantially the same method as in Examples 1-1 and 1-2.

1-4: Evaluation of enzyme activity

The cells were recovered from the cultures of the recombinant strains obtained in Examples 1-2 and 1-3 by centrifugation (4,000 rpm, 10 min). 10 mL of the recovered cells were collected and washed twice with the same amount of water. The washed microbial cells were resuspended in 100 mM phosphate buffer (pH 7.2) so that the $OD_{600}$ value was 100. Then, 100 mg of glass beads (Sigma) with a particle diameter of 0.4 to 0.6 mm were placed in a cap tube, and added by 1ml of 100 mM phosphate buffer (pH 7.2). The cells were disrupted five times for 30 seconds in a bead beater. The cell lysate liquid was centrifuged (13,000 rpm, 15 min) in refrigerated condition to obtain a supernatant as a crude enzyme solution.

The reaction solution for evaluating enzyme activity contained the substrate of Reb A (95%, Sinochem), $MgCl_2$, and UDP-Glucose. In addition, 0.1 mL of the crude enzyme solution was added to prepare a total reaction volume of 0.5 mL, and the final reaction solution was adjusted to 2 g/L of Reb A (95%, Sinochem), 3mM of $MgCl_2$, and 4 mM of UDP-Glucose. The enzyme reaction was performed on the prepared reaction solution at a temperature of 30°C, pH 7.2, and 150 rpm, and samples were taken at 3 hours and 18 hours after the start of the reaction to check the degree of reaction according to the rebound time.

The enzyme reaction solution was boiled for 5 minutes to terminate the enzyme reaction and centrifuged (13,000 rpm, 10 min).The obtained supernatant was analyzed to analyze the enzyme reaction product. Specifically, the enzyme reaction product was analyzed using HPLC-Chromatography to measure the conversion rate of the product. Since the crude enzyme solution obtained from the cell lysate of the recombinant strain showed the activity of converting Reb A substrate to Reb D, the activity was measured by using the production rate of Reb D.

Specifically, the column used for HPLC analysis of the conversion product was UG120 ($C_{18}$ 250 mm × 46 mm, 5 um 110 A particle, Shideido), and the analysis was performed at 210 nm. The mobile phase was used in a gradient with water containing 0.01% TFA (Trifluoroacetic acid, Sigma) and acetonitrile, respectively. The mobile phase was flowed at 1 mL/min and analyzed for a total of 40 minutes. The HPLC analysis conditions are shown in Table 3 below.

[Table 3]

| Time (min) | Eluent A(%) | Eluent B(%) |
|---|---|---|
| 0 | 80 | 20 |
| 10 | 80 | 20 |
| 12 | 65 | 35 |

(continued)

| Time (min) | Eluent A(%) | Eluent B(%) |
|---|---|---|
| 30 | 0 | 100 |
| 32 | 80 | 20 |
| 40 | 80 | 20 |

The HPLC analysis graph of the conversion product is shown in Table 4. The Reb D conversion rate of the enzyme that converts Reb A alone substrate to Red D shown in Table 4 below is calculated according to Formula 1 below, the relative Reb D conversion rate of the enzyme refers to the Reb D conversion rate of the TaUGT or HvUGT enzyme converted based on the 100% Reb D conversion rate of EUGT11.

[Formula 1]

Reb D Conversion rate (%) = HPLC peak area of Reb D / Total HPLC peak area

[Table 4]

| Enzyme name | Reaction time(hour) | Conversion rate of Reb D (%) | Relative conversion rate of Reb D (%) |
|---|---|---|---|
| EUGT11 | 3 | 45.8 | 100% |
| TaUGT | 3 | 43.4 | 94.8% |
| HvUGT | 3 | 32.5 | 71.0% |
| EUGT11 | 18 | 70.6 | 100% |
| TaUGT | 18 | 68.0 | 96.3% |
| HvUGT | 18 | 62.2 | 88.1% |

As shown in Table 4, the conversion rate of TaUGT in enzymatic reaction was similar to EUGT11 as a result of the initial 3-hour reaction that TaUGT converted 40-45% of Reb A to Reb D, and had an improved activity of about 30% higher than HvUGT (30-35% conversion).
In addition, as a result of analyzing the Reb D content produced in the 18-hour enzyme reaction solution, the order of conversion rates of the three enzymes showed a similar tendency to those of the 3-hour reaction.
The nucleotide sequence of TaUGT enzyme was analyzed through the nucleotide sequence analysis service of Macrogen Co., Ltd., and the amino acid sequence was analyzed based on the nucleotide sequence. As a result, the amino acid sequence of TaUGT enzyme is shown in SEQ ID NO: 1 and the nucleotide sequence is shown in SEQ ID NO: 2..

**Example 2. Production of microorganisms expressing mutant enzyme**

1-1: Construction of vector expressing mutant enzyme

In order to prepare a mutant of TaUGT enzyme of Example 1, mutants were developed based on the existing EUGT11 gene. The conserved region was identified through sequence alignment, and the amino acid differences between the domains that function as the N-term sugar acceptor and C-term sugar donor of UGT were compared. Among them, threonine at residue 201 and valine at residue 202 (corresponding to serine and leucine in EUGT11, respectively), threonine was replaced with serine, and valine was replaced with alanine or leucine. The mutation method involved synthesizing primers at the corresponding position of TaUGT (SEQ ID NOs. 24 and 25), and performing mutant gene amplification (PCR) to replace the base sequence at the corresponding region, and cloning the amplified gene fragment into the pRS426 vector through Gibson assembly.
The resulting enzyme mutant cloned into the pRS426 vector was named TaUGT [T201S/V202L]. The sequences of primers used in this example are listed in Table 5 below.

[Table 5]

| Name | Nucleotide sequence (5'->3') | SEQ ID NO |
|---|---|---|
| Forward primer for T201S mutant or V202L mutant of TaUGT | caggtatgtcwbtagcagagcgttatttcctg | 24 |
| Reverse primer for T201S mutant or V202L mutant of TaUGT | cgctctgctavwgacatacctgatgcattctgag | 25 |

2-2: Production of microorganisms expressing mutant enzyme

In order to test the expression of the enzyme, TaUGT $^{T201S/V202L}$ cloned into the pRS426 vector was selected by transforming into the same yeast strain using the LiAc method in substantially the same manner as in Example 1-2. The transformed strain was cultured in substantially the same manner as in Example 1-2. Yeast transformed with the plasmid prepared in Example 2-1 was selected in SC (synthetic complete) medium using URA-drop out mix, and the enzyme expression was induced in the selected yeast through liquid culture.

**Example 3. Evaluation for the conversion activity of mutant enzyme**

The cells were collected from the cultures of the recombinant strains obtained in Example 2-2 by centrifugation (4,000 rpm, 10 min). 10 mL of the recovered cells were collected and washed twice with the same amount of water. The washed microbial cells were resuspended in 100 mM phosphate buffer (pH 7.2) so that the $OD_{600}$ value was 100. Then, 100 mg of glass beads (Sigma) with a particle diameter of 0.4 to 0.6 mm were placed in a cap tube, and added by 1ml of 100 mM phosphate buffer (pH 7.2). The cells were disrupted five times for 30 seconds in a bead beater. The cell lysate liquid was centrifuged (13,000 rpm, 15 min) in refrigerated condition to obtain a supernatant as a crude enzyme solution.

The reaction solution for evaluating enzyme activity contained the substrate of Reb A (95%, Sinochem), $MgCl_2$, and UDP-Glucose. In addition, 0.1 mL of the crude enzyme solution was added to prepare a total reaction volume of 0.5 mL, and the final reaction solution was adjusted to 2 g/L of Reb A (95%, Sinochem), 3mM of $MgCl_2$, and 4 mM of UDP-Glucose. The enzyme reaction was performed on the prepared reaction solution at a temperature of 30°C, pH 7.2, and 150 rpm, and samples were taken at 3 hours and 18 hours after the start of the reaction to check the degree of reaction according to the rebound time.

The enzyme reaction solution was boiled for 5 minutes to terminate the enzyme reaction and centrifuged (13,000 rpm, 10 min).The obtained supernatant was analyzed to analyze the enzyme reaction product. Specifically, the enzyme reaction product was analyzed using HPLC-Chromatography in substantially the same manner as in Example 1-4, to measure the conversion rate of the product.

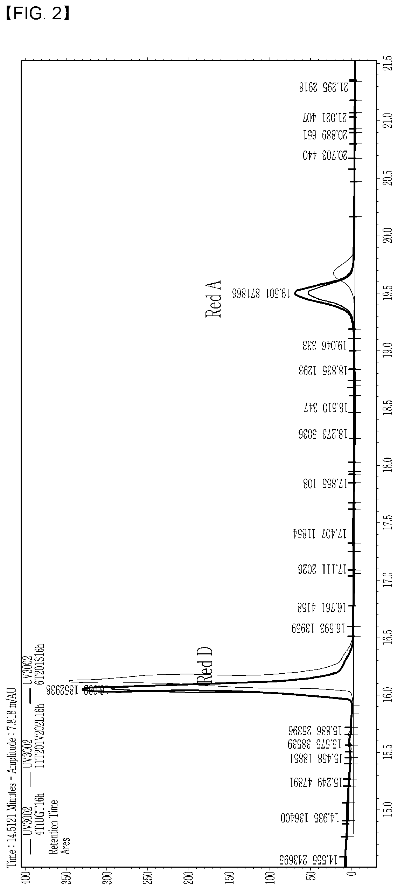

Since the crude enzyme solution obtained from the cell lysate of the recombinant strain showed the activity of converting Reb A substrate to Reb D, the activity was measured by using the production rate of Reb D, the HPLC analysis graph is shown in Fig. 2.

The Reb D conversion rate of the enzyme converting Reb A as a single substrate to Red D shown in Table 6 below, is calculated according to Formula 1 below, and the relative Reb D conversion rate of the enzyme refers to the Reb D conversion rate of TaUGT mutant enzyme, which is converted based on 100% of Reb D conversion rate of the wild type enzyme.

[Formula 1]

Reb D Conversion rate (%) = HPLC peak area of Reb D / Total HPLC peak area

An enzymatic reaction was performed for 18 hours using the reaction solution containing the TaUGT mutant enzyme and Reb A substrate, and the obtained conversion product was analyzed by HPLC analysis. The results of the activity analysis of TaUGT mutant enzymes using the HPLC analysis method are shown in Table 6 below and Fig. 2. Fig. 2 is an HPLC graph analyzing the reaction product of Reb D conversion reaction using a mutant enzyme of TaUGT.

[Table 6]

| Enzyme name | Conversion rate of Reb D(%) | Relative conversion rate of Reb D (%) |
|---|---|---|
| TaUGT (wild type) | 68.0 | 100% |
| T201S | 75.5 | 110.3% |

(continued)

| Enzyme name | Conversion rate of Reb D(%) | Relative conversion rate of Reb D (%) |
|---|---|---|
| T201S/V202L | 86.3 | 129.1% |

**Example 4. Conversion reaction using mixed substrates**

In the case of evaluation of enzyme activity using Reb A in Example 3, the conversion from Reb A to only Reb D clearly evaluated the enzyme activity. However, since mixed substrates were used in actual industrial production, a conversion reaction was performed on a mixed substrates containing stevioside and Reb A to evaluate the applicability of the enzyme tor industrial purposes using mixed substrates. That is, when mixed substrates are used, a reaction is performed to convert stevioside to Reb E, and Reb A to Reb D.

To evaluate the enzyme activity on mixed substrates, the cells of the recombinant strain obtained in Example 2-2 were resuspended in 100 mM phosphate buffer (pH 7.2) so that the OD600 value was 100. 100 mg of glass beads (Sigma) with a particle diameter of 0.4 to 0.6 mm were placed in a cap tube, and 1 ml of 100 mM phosphate buffer (pH 7.2) was added. The cells were disrupted five times for 30 seconds in a bead beater. The cell lysate liquid was centrifuged (13,000 rpm, 15 min) in refrigerated condition to obtain a supernatant, which was used as a crude enzyme solution.

The reaction solution for evaluating enzyme activity contained the reaction substrates of RA40 (Reb A (rebaudioside A: STE (stevioside) = 40:60, Sinochem), $MgCl_2$, and UDP-Glucose. In addition, 0.2 mL of the crude enzyme solution of Example 3 was added to prepare a total reaction volume of 0.5 mL, and the final reaction solution was adjusted to 2 g/L of RA40 (Reb A (rebaudioside A: STE (stevioside) = 40:60, Sinochem), 3mM of $MgCl_2$, and 4 mM of UDP-Glucose.

The enzyme reaction was performed on the prepared reaction solution at a temperature of 30°C, pH 7.2, and 150 rpm, and the degree of reaction was checked at 18 hours after reaction and samples were taken at 3 hours and 18 hours after the start of the reaction to check the degree of reaction according to the rebound time. The conversion product was analyzed with HPLC analysis method in substantially same method of Example 3.

The total Reb D/E conversion rate of the enzyme converting RA40 mixed substrates to Red D/E shown in Table 7 below, is calculated according to Formula 2 below, and the total Reb D/E relative conversion rate of the enzyme refers to the total Reb D/E conversion rate of TaUGT enzyme, which is converted based on 100% of total Reb D/E conversion rate of EUGT11 enzyme.

[Formula 2]

Reb D/E Conversion rate (%) = HPLC peak area of Reb D/E / Total HPLC peak

area

[Table 7]

| Enzyme name | Reaction time (hour) | Total conversion rate of Reb D/E(%) | Total relative conversion rate of Reb D/E (%) |
|---|---|---|---|
| TaUGT (wild type) | 18 | 78.0 | 100 |
| TaUGT (T201S) | 18 | 83.0 | 106 |
| TaUGT (T201S/V202L) | 18 | 89.2 | 114 |

As a result of the above experiment, the mutant enzyme in which Threonine at 201 residue was replaced with Serine had a relative conversion rate of 106% based on the wild type enzyme, and the double mutant enzyme containing both the substitution of Serine for Threonine at 201 residue and the substitution of Leucine for Valine at 202 residue had increased to 114 % of relative conversion rate based on the wild-type enzyme. Accordingly, the residues whose activity was improved through mutation were identified and the mutant enzymes with increased activity were obtained.

**Example 5. Preparation of fusion enzyme of TaUGT_SUS and its activity evaluation**

5-1: Preparation of recombinant yeast producing fusion enzyme of TaUGT_SUS

In order to supply the glucose donor (UDP-Glucose) required to perform the glycotransferase reaction of steviol glycosides using the TaUGT_T201S/V202L (TaUGTm) mutant enzyme which was obtained to have improved activity prepared in Example 3, the mutant enzyme was prepared as a fusion protein with sucrose synthase (SUS1, derived from *Arabidopsis thaliana*). The nucleotide sequence encoding the TaUGT_T201S/V202L (TaUGTm) mutant enzyme was the nucleotide sequence of SEQ ID NO: 30 in Table 1, and the nucleotide sequence encoding the sucrose synthase (SUS1, derived from Arabidopsis thaliana) was the nucleotide sequence of SEQ ID No: 5 in Table 1.

The preparation of the fusion enzyme was obtained by using a linker (GGGGSG, SEQ ID NO. 8) with synthesizing primers as a connecting amino acid sequence between TaUGTm and SUS, and each gene was obtained by amplifying the corresponding region according to nucleotide sequence amplification (PCR), with synthesizing primers (SEQ ID NO. 26, SEQ ID NO. 27) at the corresponding position.

After performing a gene amplification reaction (PCR), the amplified gene fragment was cloned into the pRS426 vector through Gibson assembly to produce pRS426 TaUGTm_SUS. The amino acid sequence of the SUS enzyme protein used is shown in SEQ ID NO: 4, the nucleotide sequence encoding the enzyme protein is shown in SEQ ID NO: 5, and the amino acid sequence of the fusion enzyme protein is shown in SEQ ID NO: 6. In addition, the linker sequence used to prepare the fusion protein has GGGGSG (SEQ ID NO: 8), and the amino acid and nucleotide sequences of the SUS1 enzyme protein and information on the primers used are shown in Table 8 below.

[Table 8]

| name | sequence (5'->3') | SEQ ID NO |
|---|---|---|
| Forward primer for TaUGT(mutant) | catccaaaaaaaaagtaagaattgatggacgacgggtcttctag | 26 |
| Reverse primer for TaUGT(mutant)_linker | tccactaccaccgccaccttctttataggaccttagctgttgg | 27 |
| Forward primer of Linker_SUS | ggtggcggtggtagtggaatggctaacgccgaacgtatg | 28 |
| Reverse primer of SUS | ctaattacatgatatcgacaattactcggcagccagagggac | 29 |

In the same manner as Example 1-2, the constructed expression vector for the fusion enzyme was transformed into yeast to obtain recombinant yeast expressing the fusion enzyme.

5-2: Culture of recombinant yeast and enzyme preparation

The transformed strain was cultured in substantially the same manner as in Example 1-2. Yeast transformed with the plasmid prepared in Example 2-1 was selected in SC (synthetic complete) medium using URA-drop out mix, and enzyme expression was induced in the selected yeast through liquid culture.

In substantially the same manner as in Example 3, the cells were collected from the culture of the recombinant strain using centrifugation, and the cells were disrupted with a glass beader. The cell lysate was centrifuged (13,000 rpm, 15 min) in refrigerated condition to obtain a supernatant as a crude enzyme solution.

**5-3: Evaluation for the conversion activity of fusion enzyme**

The raw reaction solution for conversion resection was prepared by mixing the reaction substrates of RA40 (Reb A (rebaudioside A: STE (stevioside) = 40:60, Sinochem), $MgCl_2$, UDP and sucroase, and adding 0.1 mL of the crude enzyme solution of TaUGTm_SUS fusion enzyme obtained in Example 5-2 to prepare a total reaction volume of 0.5 mL, and the final reaction solution was adjusted to 10 g/L of RA40 (Reb A (rebaudioside A: STE (stevioside) = 40:60, Sinochem), 1 mM of $MgCl_2$, and 1 mM of UDP, and 125 mM of sucrose. An enzyme reaction was performed on the prepared reaction solution at a temperature of 30°C, pH 7.2, and 150 rpm, and the enzyme conversion was evaluated by reacting for 20 hours.

In substantially the same way as Example 1-4, the conversion product of the reaction solution was analyzed by HPLC analysis. It was verified that the fusion enzyme converted RA40 (Reb A: STE = 40:60) to Reb D/E with supplying UDP_glucose from UDP. STE refers to stevioside.

About 33% by weight of the substrate was converted to Reb D/E through the initial conversion reaction of the fusion enzyme, on the basis of to 100% by weight of the substrate before the reaction. Through the conversion reaction of the mixed substrates (RA40 (Reb A: STE = 40:60)), Reb D (including D derivatives) and Reb E were converted to 21.7% and 12.1%, respectively. The activity of the fusion enzyme that produced UDP_Glucose from sucrose and saccharification of the substrate using the UDP_glucose were identified. It was verified that it was possible to improve the conversion reaction of the enzyme by optimizing the amount of substrate and reaction conditions.

When using a fusion enzyme to convert a substrate equivalent to about 10 mM by using 10 g/L substrate of RA40 (Reb A: STE = 40:60), more than 10 to 20 mM of UDP_Glucose is required. Therefore, when converting a substrate in a large amount of at high concentration, an excess amount of expensive UDP_Glucose is required. When carrying out an enzyme reaction using a high concentration of substrate, it is not preferable, because an excessive amount of UDP_Glucose must be used and excess UDP_Glucose inhibits enzyme activity.

According to this experiment, when using the TaUGT_SUS fusion enzyme, it is possible to perform conversion using 1/10 of UDP amount for the substrate, and to perform reaction using UDP at an amount lower than 1/100 of UDP amount for RA40 substrate. It was verified that the conversion reaction using a fusion enzyme could make an economical enzyme conversion, and that the steviol glycosides could be produced through a reaction using high-concentration of substrate.

**Example 6. Preparation of microorganism expressing UGT and SUS enzymes**

6-1: Construction of a vector introduced by enzyme gene

In order to produce recombinant yeast that produces all three types of TaUGTm, SrUGT76G1, and Sucrose synthase to perform the conversion reaction to Rebaudioside M, a vector into which the above genes were introduced was first prepared.

Specifically, using substantially the same production method, primer, and linker as in Example 5-1, pRS423_UGT76G1_GmSUS as a vector for the fusion enzyme of UGT76G1 and GmSUS, and pRS426_TaUGTm_GmSUS vector as a vector for the fusion enzyme of TaUGTm and GmSUS were prepared. The TaUGTm is the same as in Example 5, TaUGT_T201S/V202L (TaUGTm), except that sucrose synthase is an enzyme derived from *Glycine max* (soybean), to have nucleotide sequence of SEQ ID NO: 34 in Table 1. In addition, SrUGT76G1 used above is a UDP-glycosyltransferase derived from *Stevia rebaudiana* (stevia plant), to have nucleotide sequence encoding a fusion enzyme using nucleotide sequence shown in SEQ ID NO: 32 of Table 1, and is introduced as a nucleotide sequence encoding a fusion protein.

6-2: Preparation of host cells

Yeast, which is a host cell for introducing the prepared vector, was used to prepare *Saccharomyces cerevisiae* CENPK2-1c (Euroscarf)△ga180△SUC2 by removing SUC2 gene encoding the invertase enzyme to prevent sucrose degradation

Specifically, removal of the SUC2 gene was performed using the yeast homologous recombination method, and each 500-bp DNA sequence at upstream and downstream regions of the SUC2 gene was amplified through PCR using Q5 DNA polymerase (NEB) under conditions of 98°C (1m). -[98°C(10s)-55°C(30s)-72°C(5m)] 30 cylces-72°C(5m)-16°C(storage), and information on the upstream and downstream sequences are shown in Table 9 below.

[Table 9]

| Name | Nucleotide sequence (5' -> 3') | SEQ ID NO |
|------|--------------------------------|-----------|
| SUC2 upstream sequence 446-bp | AAATTATCCGGGGGCGAAGAAATACGCGTAGCGTTAATCGACCCCACGTCC AGGGTTTTTCCATGGAGGTTTCTGGAAAAACTGACGAGGAATGTGATTATAA ATCCCTTTATGTGATGTCTAAGACTTTTAAGGTACGCCCGATGTTTGCCTATT ACCATCATAGAGACGTTTCTTTTCGAGGAATGCTTAAACGACTTTGTTTGACA AAAATGTTGCCTAAGGGCTCTATAGTAAACCATTTGGAAGAAAGATTTGACG ACTTTTTTTTTTTGGATTTCGATCCTATAATCCTTCCTCCTGAAAAGAAACATA TAAATAGATATGTATTATTCTTCAAAACATTCTCTTGTTCTTGTGCTTTTTTTTTT ACCATATATCTTACTTTTTTTTTTTCTCTCAGAGAAACAAGCAAAACAAAAAGCT TTTCTTTTCACTAACGTATATG | 35 |

(continued)

| Name | Nucleotide sequence (5' -> 3') | SEQ ID NO |
|------|-------------------------------|-----------|
| SUC2 downstream sequence 358-p | GGTTATAAAACTTATTGTCTTTTTTATTTTTTTCAAAAGCCATTCTAAAGGGCTTTAGCTAACGAGTGACGAATGTAAAACTTTATGATTTCAAAGAATACCTCCAAACCATTGAAAATGTATTTTTATTTTTATTTTCTCCCGACCCCAGTTACCTGGAATTTGTTCTTTATGTACTTTATATAAGTATAATTCTCTTAAAAATTTTTACTACTTTGCAATAGACATCATTTTTTCACGTAATAAACCCACAATCGTAATGTAGTTGCCTTACACTACTAGGATGGACCTTTTTGCCTTTATCTGTTTTGTTACTGACACAATGAAACCGGGTAAAGTATTAGTTATGTGAAAATTT | 36 |

After performing the PCR amplification, each amplified DNA fragment was connected using the PCR overlap extension method. The connected DNA fragment was cloned into pRS26 vector and was transformed to yeast using the LiAc method, and the obtained transformed strain were selected for strains with the deleted SUC2 gene through colony PCR.

6-3: Preparation of transformed yeast

In substantially the same manner as in Example 5, the pRS423_UGT76G1_GmSUS and pRS426_TaUGTm_GmSUS vectors prepared in Example 6-1 were transformed into *Saccharomyces cerevisiae* CENPK2-1c (Euroscarf) Δga180ΔSUC2 prepared in Example 6-2 by the LiAc method..

The transformed strain was cultured in substantially the same manner as in Example 1-2. Yeast transformed with the plasmid prepared in Example 2-1 was selected on SC (synthetic complete) medium using URA-drop out mix.

Through 5L fermentation culture of the selected transformed yeast, three types of enzymes were produced within the cells. The volume of the production medium used in the culture was 2L, the composition of the production medium used was as shown in Table 10 below, a feeding solution with the composition shown in Table 11 below was additionally added during the fermentation culture, and information on vitamins 1000x and Trace metal 1000x in the production medium information is shown in Tables 12 and 13 below, respectively.

[Table 10]

| Ingredient | Concentration (g/L) | Weight (g) |
|-----------|---------------------|------------|
| Glucose | 30 | 60 |
| $MgSO_4 \cdot 7H_2O$ | 0.5 | 1 |
| $(NH_4)_2SO_4$ | 5 | 10 |
| $KH_2PO_4$ | 3 | 6 |
| Trace metals (1000x) | 2mL | 4mL |
| Vitamins (1000x) | 1mL | 2mL |

[Table 11]

| Ingredient of Feeding Solution | Concentration (g/L) | Weight (g) |
|--------------------------------|---------------------|------------|
| Glucose | 500 | 500 |
| $MgSO_4 \cdot 7H_2O$ | 6.25 | 6.25 |
| $(NH_4)_2SO_4$ | 62.5 | 62.5 |
| $KH_2PO_4$ | 18.75 | 18.75 |
| Vitamins (1000x) | 6.25mL | 6.25mL |

(continued)

| Ingredient of Feeding Solution | Concentration (g/L) | Weight (g) |
|---|---|---|
| Trace Elements (1000x) | 12.5mL | 12.5mL |

[Table 12]

| Ingredient | Concentration (g/L) |
|---|---|
| biotin | 0.05 |
| p-amino benzoic acid | 0.2 |
| Nicotinic acid | 1 |
| Ca-pantothenate | 1 |
| pyridoxine-HCl | 1 |
| thiamine-HCl | 1 |
| myo-inositol | 25 |

[Table 13]

| Ingredient | Concentration (g/L) |
|---|---|
| EDTA | 19 |
| $ZnSO_4 \cdot 7H_2O$ | 4.5 |
| $MnCl_2 \cdot 2H_2O$ | 0.84 |
| $CoCl_2 \cdot 6H_2O$ | 0.3 |
| $CuSO_4 \cdot 5H_2O$ | 0.3 |
| $Na_2MoO_4 \cdot 2H_2O$ | 0.4 |
| $FeSO_4 \cdot 7H_2O$ | 3 |
| $CaCl_2 \cdot 2H_2O$ | 4.5 |
| $H_3BO_3$ | 1 |
| KI | 0.1 |

Yeast cells transformed with the genes of three types of enzymes were cultured to ensure a final OD600 value of approximately 120/mL after culture. Then, the culture medium and cells were separated through centrifugation (4,000 rpm, 4 °C, 10 minutes).

6-4: Conversion reaction using crude enzyme solution

The separated cells were resuspended in 500 mL of 50mM Sodium phosphate buffer (pH 7.2), the cells were disrupted using a high pressure disperser (GEA Lab Homogenizer Panda PLUS 2000), and the disrupted cells were used directly as an enzyme source without a separate centrifugation process.

The reaction solution for the conversion reaction was prepared by dissolving 50g/L of stevia extract RA60_SG95 (Sinochem, China), 750mM sucrose, 0.5mM uridine diphosphate glucose (UDP) and 3mM MgCl2 in 500mL of 50mM sodium phosphate buffer (pH 7.2). The RA60_SG95 refers to the stevia extract that contains 95 (w/v)% of steviol glycosides as an active ingredient, and that 60 (w/w)% of Reb A content, and 40 (w/w)% of the stevioside content are contains based on 100% by weight of steviol glycosides.

The cell lysate containing the enzyme and the raw materials (reaction solution) prepared above for the conversion reaction were mixed and stirred at 30°C for 24 hours to proceed with the Rebaudioside M conversion reaction. After the conversion reaction, the reaction solution was boiled for 5 minutes to stop the reaction and then centrifuged (13,000 rpm, 10 minutes). The supernatant obtained through centrifugation was analyzed to verify the product.

The product was analyzed qualitatively and quantitatively using HPLC to measure content of the produced steviol glycosides. The column used in HPLC was UG120 (C18 250mm x 46mm, 5um 110 A particle, Shideido), and analysis was performed at 210nm. The mobile phase was performed in a gradient using water containing 0.01% TFA (Trifluoroacetic acid, Sigma) and 100% acetonitrile (ACN), respectively. The mobile phase was flowed at 1 mL/min and analyzed for a total of 40 minutes. The HPLC analysis conditions are shown in Table 14 below.

[Table 14]

| Time (minute) | Eluent A (%) 0.01% TFA | Eluent B (%) 100% ACN |
|---|---|---|
| 0 | 80 | 20 |
| 10 | 80 | 20 |
| 12 | 65 | 35 |
| 30 | 0 | 100 |
| 32 | 80 | 20 |
| 40 | 80 | 20 |

The above analysis results show that the retention times obtained from HPLC analysis are 15.3 minutes for STE and 15.1 minutes for Reb A in the substrate, and 10.6 minutes for Reb E, 14.7 minutes for Reb I, 10.9 minutes for Reb D and 11.6 minutes for Reb M.

**Example 7. Test of pH condition for conversion reaction**

The reaction pH of the Rebaudioside M conversion reaction from stevia extract was optimized using the cell lysate (crude enzyme solution) of the transformed yeast cells producing the three enzymes obtained in Example 6-4.

Specifically, the reaction solution was prepared in the same manner as the raw materials (raw reaction material solution) for the conversion reaction prepared in Example 6, but the pH of the reaction solution in Example 6 was adjusted to 5.8, 6.0, 6.2 or 6.5 using 5N NaOH.

Using the pH-adjusted reaction solution, Rebaudioside M conversion reaction was performed in substantially the same manner as in Example 6, centrifugation was performed to obtain a supernatant, and the steviol glycosides content of the reaction product was analyzed using HPLC analysis. The analysis results are shown in Table 15 below. The numerical values in Table 15 below refer to the composition of each steviol glycoside in % by weight based on 100% by weight of the produced steviol glycosides.

[Table 15]

| pH condition | STE | Reb A | Reb E | Reb I | Reb D | Reb M |
|---|---|---|---|---|---|---|
| 5.8 | 0.0 | 12.7 | 0.0 | 8.7 | 46.3 | 32.3 |
| 6 | 0.0 | 3.1 | 0.0 | 10.4 | 26.1 | 60.4 |
| 6.2 | 0.0 | 1.2 | 0.7 | 12.1 | 20.3 | 65.7 |
| 6.5 | 0.0 | 7.1 | 0.0 | 60.0 | 4.5 | 28.4 |

As a result of analyzing the steviol glycosides content of the product, the glycolysis site in steviol performed by glycosyltransferase was located at carbon atoms 13 and 19 of steviol. It was confirmed that the glycosylation rate at C19 was fast when pH is 6.0 or higher, and the glycosylation rate at C13 was fast when pH was 6.0 or less in the actual reaction.

In the enzymatic reaction using the three types of enzymes of TaUGTm, SrUGT76G1, and sucrose synthase, the optimum pH conditions act in a complementary manner, resulting in the highest production of Rebaudioside M from the stevia extract between pH 6.0 and 6.2.

**Example 8. Test of temperature condition for conversion reaction**

The reaction temperature of the Rebaudioside M conversion reaction from stevia extract was optimized using the cell lysate (crude enzyme solution) of the transformed yeast cells producing the three enzymes obtained in Example 6-4.

Specifically, the reaction solution was prepared in the same manner as the raw materials (raw reaction material solution) for the conversion reaction prepared in Example 6, but the pH of the reaction solution as adjusted to 6.0 using 5N NaOH, and the cell lysate containing the enzyme and the raw materials (reaction solution) prepared in Example 6 were mixed and stirred at 30°C, 35°C, 40°C, or 45°C for 24 hours, to proceed with the Rebaudioside M conversion reaction under other conditions of substantially the same manner as in Example 6. The supernatant was obtained through centrifugation and the steviol glycoside contents were analyzed through HPLC method.

The analysis results of steviol glycosides content according to the reaction time are shown in Table 16 below.

The content of Reb M was calculated as a conversion rate according to Formula 3 below, using the area of the third peak corresponding to Reb M (g/L) in the HPLC peak.

[Formula 3]

$$\text{Content of Reb M (g/L)} = ([\text{HPLC area}] - 6324]\} / 938442$$

The numerical values in Table 16 below refer to the content of Reb M(g/L).

[Table 16]

| Reaction temperature (°C) | 0 hour | 1 hour | 2 hour | 4 hour | 6 hour | 8 hour | 24 hour |
|---|---|---|---|---|---|---|---|
| 30 | 0 | 8.7 | 23.4 | 46.6 | 68.5 | 85.2 | 92.3 |
| 35 | 0 | 13.2 | 28.4 | 55.3 | 78.7 | 94.1 | 98.4 |
| 40 | 0 | 17.6 | 35.9 | 60.7 | 65.4 | 70.1 | 68.6 |
| 45 | 0 | 21.9 | 48.2 | 56.5 | 58.1 | 61.3 | 59.9 |

As a result of analyzing the Rebaudioside M conversion reaction according to reaction temperature, the initial conversion rate increased as the reaction temperature increased, but enzyme deactivation occurred quickly, and conversion rate of Rebaudioside M was confirmed to be highest at 35°C finally.

**Example 9. Test of sucrose concentration for conversion reaction**

In the conversion reaction of Rebaudioside M from stevia extract using the cell lysate (crude enzyme solution) of the transformed yeast cells producing the three enzymes obtained in Example 6-4, the inhibition degree on the conversion reaction was tested according to the concentration of sucrose that was used as a substrate for supplying glucose in the glycosylation.

The theoretical amount of UDP-glucose required for conversion to Rebaudioside M for 50g/L of stevia extract is expected to be about 320mM. However, since in the actual conversion reaction, about 200mM of sucrose is decomposed by other enzymes in the cell lysate, optimization of the sucrose concentration used in the conversion reaction began at least 500mM.

Specifically, the reaction solution was prepared in the same manner as the raw materials for the conversion reaction prepared in Example 6, but the raw reaction material was prepared by using 600, 80 1000 or 1200 mM of sucrose concentration instead of 750mM of sucrose concentration used in the conversion reaction of Example 6, and adjusting the pH of reaction solution to 6.0 using 5N NaOH. The prepared raw reaction material was mixed with the cell lysate (crude enzyme solution) of the transformed yeast cells producing the three enzymes obtained in Example 6-4, and stirred at 35°C, to proceed with the Rebaudioside M conversion reaction under other conditions of substantially the same manner as in Example 6. The supernatant was obtained through centrifugation and the steviol glycoside contents were analyzed through HPLC method.

The analysis results of steviol glycosides content according to the sucrose concentrations are shown in Table 17 below. The numerical values in Table 17 below refer to the conversion rate of Reb M(%).

【Formula 4】

Reb M Conversion rate (%) = HPLC peak area of Reb M / Total HPLC peak area

[Table 17]

| Sucrose concentration (mM) | Conversion rate of Reb M(w/w%) |
|---|---|
| 600 | 63.2 |
| 800 | 60.3 |
| 1000 | 72.8 |
| 1200 | 34.6 |

From the above experimental results, it was confirmed that Rebaudioside M had the highest conversion rate at a concentration of 1000mM of sucrose included in the raw material for the conversion reaction, and at concentrations higher than this, inhibition of the sucrose synthase enzyme occurred, resulting in decrease of the overall conversion rate.

**Example 10. Conversion reaction of Rebaudioside M from high concentration stevia extract**

Based on the reaction conditions verified in Examples 6 to 9, Rebaudioside M conversion rates performed from high-concentration stevia extract were compared.
The overall conversion reaction scheme for the production of Rebaudioside M using three types of enzymes according to Example 6 is shown in Reaction scheme 1 below.

[Reaction Scheme 1]          SUS: Sucrose + UDP + [H$^+$] → Fructose + UDP-glucose UGT: Steviol glycoside + UDP-glucose → Steviol glycoside-[glucose] + UDP + [H$^+$]

In order to proceed with the experiment under the optimized reaction conditions verified in Examples 6 to 9, four substrate concentrations of 25, 50, 75 or 100 g/L of raw reaction materials were prepared instead of the RA60_SG95 (stevia extract) substrate concentration of 50 g/L used in the preparation of raw material for the conversion reaction in Example 6, and adjusted to 1000mM of the sucrose concentration and 6.0 of pH.
The prepared reaction raw materials were mixed with the cell lysate (crude enzyme solution) of the transformed yeast cells producing the three enzymes obtained in Example 6-4, and stirred at 35°C. Rebaudioside M conversion reaction was performed under other conditions of substantially the same manner as in Example 6, the supernatant was obtained by centrifugation, and the steviol glycosides content of the product and the content ratio of components in the steviol glycosides of the product were analyzed using HPLC analysis.
The production amount of Rebaudioside M(g/L) obtained from the conversion reaction is shown in Table 18 below. In addition, the results of analysis of the content ratio of components in the steviol glycosides of the product are shown in Table 19 and Fig. 1, and the numerical values shown in Table 19 below indicate the content ratio (% by weight) of each steviol glycoside, based on 100% by weight of the sum of STE, Reb A, Reb E, Reb I, Reb D, and Reb M.

[Table 18]

| Substrate concertation (g/L) | Production amount of Reb M (g/L) |
|---|---|
| 25 | 34.1 |
| 50 | 67.8 |
| 75 | 96.8 |
| 100 | 125.5 |

[Table 19]

| Substrate concertation (g/L) | STE | Reb A | Reb E | Reb I | Reb D | Reb M |
|---|---|---|---|---|---|---|
| 25 | 0 | 0 | 0 | 4.6 | 0 | 95.4 |
| 50 | 0 | 0 | 0 | 1.6 | 3.6 | 94.8 |
| 75 | 0 | 0 | 0 | 2.3 | 7.5 | 90.2 |
| 100 | 0 | 0 | 0 | 2.8 | 9.5 | 87.7 |

As a result of performing the conversion reaction using a high-concentration steviol extract under the reaction temperature, reaction pH, and sucrose concentration conditions verified in Examples 6 to 9, the absolute production amount of Rebaudioside M increased as the concentration of the substrate of steviol extract increased.

As shown in Table 19, at all four substrate concentrations of 25, 50, 75 or 100 g/L, the Reb M production rate was the highest among the six steviol glycosides. In addition, although the content of Rebaudioside M in all steviol glycosides slightly decreased as the substrate concentration increased, a high conversion rate of 87.7% was achieved at a concentration of 100 g/L which was the minimum substrate concentration for industrial mass production, thereby showing that a high conversion rate of Reb M was obtained in high-concentration of stevia extract.

In addition, it was confirmed that the absolute production amount of Rebaudioside M through the conversion process increased as the substrate concentration increased. Therefore, even though the content ratio of Rebaudioside M in the steviol glycosides of the product was slightly lower, its absolute production amount increased, confirming that the recombinant microorganism of the present invention had very excellent characteristics under conditions for industrial mass production.

It was confirmed that Rebaudioside I in the reaction product using all four substrate concentrations of 25, 50, 75, or 100 g/L was contained, confirming that the inclusion of Rebaudioside I as a by-product was unavoidable.

Through the above results, it was confirmed that when converting stevia extract to steviol glycosides, mass production in terms of Rebaudioside M conversion rate could be achieved with overcoming the low conversion rate and low production rate that occurred in the conventional Rebaudioside M conversion reaction using high concentration of stevia extract.

## Example 11. Freeze-drying of microorganisms expressing UGT and SUS enzymes

11-1: Preparation of transformed yeast cells producing UGT and SUS enzymes

Cells of transformed microorganisms expressing UGT and SUS enzymes were prepared and obtained by the methods of Examples 6-1, 6-2, and 6-3 above.

11-2: preparation of Freeze-dried microorganisms

In order to increase substrate permeability into microbial cells and increase storage stability at room temperature, the microbial cells prepared in Example 11-1 were freeze-dried. Freeze-drying was performed by rapidly freezing at -40°C while maintaining vacuum, and then freeze-drying for 32 hours using a freeze-drying device.

## Example 12: Evaluation of conversion activity of freeze-dried cells

In a whole-cell reaction using the freeze-dried cells prepared in Example 11-2, the conversion reaction of Reb M was performed in the reaction substrate to evaluate the conversion activity of the freeze-dried cells.

As a control group, transformed microbial cells collected after culturing in Example 11-1 without freeze-drying were prepared. Cells that were not subjected to freeze-drying were prepared.

In order to evaluate the enzyme activity of the cells, 1 mL of reaction raw material solution was prepared by adding 1 g/L of substrate RA60 (95%, Sinochem), 1mM of UDP, and 250mM of sucrose to double distilled water (DDW). The freeze-dried whole cells prepared in Example 11-2 were added to the reaction raw materials. The freeze-dried cells (experimental group) prepared in Example 11-2 or non-freeze-dried cells(control group) prepared in Example 11-1 were added to the reaction raw materials, so as to adjust 9.2 g/L, respectively.

The conversion reaction was performed on the prepared reaction solution at a temperature of 30°C, pH 7.0, and 150 rpm, and after starting the reaction for 1 hour, the conversion reaction was terminated by boiling the whole-cell reaction solution at 100°C for 5 minutes, and the supernatant was obtained through centrifugation ( 13,000 rpm, 10 min) to analyze the whole-cell reaction product.

Analysis of the whole-cell reaction product was performed using HPLC-Chromatography to measure the conversion rate of steviol glycosides contained in the product. Specifically, the HPLC analysis method for the conversion product was performed in substantially the same as the method in Example 6-4. The recombinant microorganism converted the Reb A substrate into Reb D and Reb D into Reb M, so as to measure a production ratio of Reb D and Reb M.

As a result of the above analysis, the conversion rates from RA60 substrate to Red D and Reb M are shown in the table below. The Reb D conversion rate and Reb M conversion rate were calculated according to Equations 1 and 4 below, respectively.

[Formula 1]

$$\text{Reb D Conversion rate (\%)} = \text{HPLC peak area of Reb D /total HPLC peak area}$$

[Formula 4]

$$\text{Reb M Conversion rate (\%)} = \text{HPLC peak area of Reb M / Total HPLC peak area}$$

[Table 20]

| Microbial cells | Reb D conversion rate(%) | Reb M conversion rate(%) |
|---|---|---|
| Freeze-dried cell in Example 11-2 | 47.5 | 14.7% |
| Non-freeze-dried cell in Example 11-1 | 0 | 0 |

As a result of calculating Reb D conversion rate and Reb M conversion rate, Reb D and Reb M were not produced in the conversion reaction using non-lyophilized cells, but Reb D and Reb M were produced in the conversion reaction using freeze-dried cells. Additionally, the conversion rate of Reb M was 14.7% and the conversion rate of Reb D was 47.5%. It was confirmed that freeze-dried whole cells were able to produce Reb D and M. In Example 10, when a cell lysate solution (crude enzyme solution) containing three types of enzymes was used, the content ratio of the Reb M component in the steviol glycoside of the product was 80% or more, but in this experiment of the whole-cell reaction, the cell wall permeability of the substrate was low, so the Reb M production rate tended to be relatively low.

**Example 13: Stability of freeze-dried cells**

To evaluate the stability of the freeze-dried microorganism obtained in Example 11-2, it was tested whether the enzyme activity was maintained after being stored at room temperature (25°C) for 9 weeks. In order to test maintenance of enzyme activity, enzyme activity was measured once a week in substantially the same manner as in Example 12 above.

As a result of testing the enzyme activity of the freeze-dried microbial cells for the above storage period, it was confirmed that the enzyme activity was maintained for the entire test period of 9 weeks, thereby suggesting that the cells could be stored at room temperature. Specifically, when the freeze-dried cells were stored for 9 weeks, it was confirmed that the enzyme activity of the cells was maintained at more than 97% based on 100% activity at the start point of storage. On the other hand, the cell lysate used in the conversion reaction in Example 10 or the crude enzyme solution obtained therefrom had almost lost its enzyme activity 24 hours after cell lysing, and the remaining activity is reduced to 10% or less.

**Example 14. Optimization of whole cell conversion reaction using freeze-dried cells**

14-1: pH optimization of whole cell reactions

In order to test the optimal pH of the whole-cell reaction using freeze-dried cells, the whole-cell conversion reaction was performed in substantially the same manner as Example 12, using the freeze-dried microbial cells obtained in Example 11-2. However, 100 mM Potassium phosphate buffer was added and pH was adjusted to 6.0 to 7.4. Then, the whole-cell reaction solution was boiled at 100°C for 5 minutes to terminate the whole-cell reaction. The supernatant was obtained through centrifugation(13,000 rpm, 10 min), and was analyzed by HPLC under the HPLC conditions of Example 12 to analyze the whole-cell reaction product.

The relative conversion rate was set and expressed for the rest as relative ratios (%), when 100 for conversion rate at the highest Reb M production was set within the entire pH range, and the rest were expressed as relative ratios (%). The results are shown in the table below.

[Table 21]

| pH | Relative conversion rate (%) |
|---|---|
| 6.2 | 55 |
| 6.4 | 63 |
| 6.6 | 72 |
| 6.8 | 80 |
| 7 | 89 |
| 7.2 | 100 |
| 7.4 | 92 |

As a result of measuring the conversion rate of the whole cell reaction according to the pH conditions of the conversion reaction, as the pH increased starting from pH 6.0, the relative conversion rate increased and was the highest at pH 7.2. The optimal pH for the whole cell reaction of freeze-dried cells was confirmed to be 7.2.

14-2: Optimization of reaction temperature for whole-cell reaction

In order to verify the optimal temperature for the whole-cell reaction using freeze-dried cells, the whole-cell conversion reaction was performed in substantially the same manner as Example 12 using the freeze-dried microbial cells obtained in Example 11-2, however, the whole-cell reaction was performed at pH 7.2 by adding 100 mM potassium phosphate buffer at a temperature of 30, 33, 36, 39, 42, 45, 48, 52, or 55°C.

Then, the whole-cell reaction solution was boiled at 100°C for 5 minutes to terminate the whole-cell reaction, and supernatant was centrifuged (13,000 rpm, 10 min) and was analyzed by HPLC under the HPLC conditions of Example 12-2 to obtain the whole-cell reaction product. Within the entire temperature range, the highest Reb M production result was set as 100, and the rest were expressed as relative ratios (%). The results are shown in the table below.

[Table 22]

| Reaction temperature (°C) | Relative activity(%) |
|---|---|
| 30 | 58 |
| 33 | 72 |
| 36 | 94 |
| 39 | 95 |
| 42 | 98 |
| 45 | 100 |
| 48 | 99 |
| 52 | 94 |
| 55 | 62 |

As a result of verifying the conversion rate of the whole cell reaction according to the temperature conditions of the conversion reaction, the conversion rate increased as the temperature increased from 30°C to about 35°C, and the conversion reaction was performed stably at a higher temperature, up to about 52°C, and the highest conversion rate was shown at 45°C, confirming that the optimal temperature for the whole cell reaction of freeze-dried cells was 45°C.

**Example 15. Mass production of Reb M at high purity by using freeze-dried cells**

To test whether high concentration of Reb M could be produced using the freeze-dried cells obtained in Example 11-2, the conversion reaction using whole cells was performed.

Specifically, in order to perform the mass manufacturing process for producing the high concentration of Reb M, 2L of whole-cell reaction solution containing 75 g/L of RA60 (95%, Sinochem) as a substrate, UDP 0.3mM, Sucrose 750mM, 45g/L of freeze-dried cells and 5mM of EDTA in double distilled water (DDW) was prepared.

The pH was adjusted to 7.2 by adding 5 mM potassium phosphate buffer to the prepared reaction solution, the whole-cell reaction was performed at 45°C and 250 rpm, and the whole-cell reaction solution was boiled at 100°C for 5 minutes to terminate the whole-cell reaction. After completion, centrifugation (4000 rpm, 20 min) was performed to obtain the supernatant. HPLC analysis was performed using the supernatant under the HPLC conditions of Example 12, and the amount of produced Reb M was measured.

As a result of the above analysis, a Reb M conversion rate of 90.4% and a Reb M production amount of 97 g/L were obtained. As a result of measuring the production amount of Reb M, it was confirmed that high concentration of Reb M with a purity of 90% by weight could be obtained using freeze-dried cells, thereby making mass production of Reb M with high purity be possible. Although a small amount of substrate solution was used to evaluate the productivity of Reb M using freeze-dried cells and the production conditions were somewhat different, it was confirmed that the conversion rate was significantly increased in this experiment compared to Reb M production in Example 12.

**Claims**

1. A composition for producing steviol glycosides containing Rebaudioside M,

   which comprises a biocatalyst comprising a first uridine diphosphate-glucosyltransferase(UDP-glucosyltransferase) that has 92% or higher of amino acid sequence identity with an amino acid sequence of SEQ ID NO: 1 and transfers glucose to steviol glycoside substrate, and UGT76G1; and a reaction substrate;
   wherein Rebaudioside M is contained in 10% by weight or more of the steviol glycosides reaction product.

2. The composition of Claim 1, wherein the first UDP-glucosyltransferase comprises an amino acid sequence of SED ID NO: 1, or an amino acid sequence comprising at least a substitution of amino acid in which at least an amino acid selected from the group consisting of 201st amino acid and 202nd amino acid from N-terminus in an amino acid sequence of SEQ ID NO: 1, is substituted with at least one selected from serine and valine.

3. The composition of Claim 1, wherein the biocatalyst further includes a sucrose synthase protein.

4. The composition of Claim 3, wherein the sucrose synthase is provided as a fusion protein linked directly or through a linker with at least an enzymes selected from the group consisting of first UDP-glucosyltransferase and UGT76G1 enzyme.

5. The composition of Claim 1, wherein the Rebaudioside M is contained at 50% by weight or more of the steviol glycosides reaction product.

6. The composition of Claim 1, wherein the biocatalyst comprises at least one selected from the group consisting of the enzyme protein, cells of microorganism, cultures of the microorganism, lysates of the microorganism, and extracts of the lysate or the culture.

7. The composition for producing steviol glycosides containing Rebaudioside M of Claim 1, which comprises a biocatalyst comprising a first uridine diphosphate-glucosyltransferase(UDP-glucosyltransferase) that has 92% or higher of amino acid sequence identity with an amino acid sequence of SEQ ID NO: 1 and transfers glucose to steviol glycoside substrate, and UGT76G1; and a reaction substrate,

   wherein the biocatalyst is at least one selected from the group consisting of microbial cells of microorganism and dried product of the microbial cells, and
   wherein Rebaudioside M is contained in 10% by weight or more of the steviol glycosides reaction product.

8. The composition of Claim 7, wherein the dried product of the microbial cells has 30% or more of a total conversion rate of Reb M and Reb D.

9. The composition of Claim 7, wherein after the dried product of the microbial cells is stored at room temperature for 9 weeks, the enzyme activity of the microbial cells is 90% or higher of the Rebaudioside M conversion activity of the microbial cells immediately after drying.

10. The composition of Claim 7, wherein the freeze-dried cells are dried product that is freeze-dried at a temperature of -60 to 40°C and under reduced pressure conditions.

11. The composition of Claim 7, wherein the composition further comprises ethylenediaminetetraacetic acid.

12. The composition of Claim 11, wherein the concentration of ethylenediaminetetraacetic acid is 1 to 20mM.

13. The composition of Claim 1, wherein the reaction substrate includes at least one steviol glycosides selected from the group consisting of stevioside and Rebaudioside A.

14. The composition of Claim 1, wherein the reaction substrate is provided in an amount of 15 to 150 g/L.

15. The composition of Claim 1, wherein the biocatalyst is a strain of *Escherichia coli,* a strain of the genus Saccharomyces, or a strain of the genus Pichia.

16. The composition of Claim 1, wherein the microorganism is a microorganism of the genus Saccharomyces in which SUC2 gene encoding the invertase enzyme is deleted.

17. The composition of Claim 1, wherein the composition further comprises $MgCl_2$ or $MnCl_2$.

18. The composition of Claim 1, further comprising at least one selected from the group consisting of uridine diphosphate (UDP) and sucrose.

19. The composition of Claim 18, comprising sucrose at a concentration of 500 to 2,000 mM.

20. The composition of Claim 1, wherein the reaction substrate is in pH 4 to 9.

21. A method for producing steviol glycosides containing Rebaudioside M, comprising reacting a reaction substrate containing at least one selected from the group consisting of stevioside and Rebaudioside A, with a biocatalyst containing a first uridine diphosphate-glucosyltransferase and UGT76G1,

   wherein the first uridine diphosphate-glucosyltransferase has an amino acid sequence identity of 92% or higher with an amino acid sequence of SEQ ID NO: 1, and has UDP-glycosyltransferase activity transferring glucose to a steviol glycoside substrate, and
   wherein the Rebaudioside M is contained at 10% by weight or more of the steviol glycosides reaction product.

22. The method of Claim 21, wherein the biocatalyst comprises at least one selected from the group consisting of the enzyme protein, cells of microorganism, cultures of the microorganism, lysates of the microorganism, and extracts of the lysate or the culture.

23. The method for producing steviol glycosides containing Rebaudioside M of Claim 21, comprising reacting the reaction substrate containing at least one selected from the group consisting of stevioside and Rebaudioside A, with a biocatalyst containing a first uridine diphosphate-glucosyltransferase and UGT76G1,

   wherein the first uridine diphosphate-glucosyltransferase has an amino acid sequence identity of 92% or higher with an amino acid sequence of SEQ ID NO: 1, and has UDP-glycosyltransferase activity that transfers glucose to a steviol glycoside substrate, and
   wherein the biocatalyst is at least one selected from the group consisting of microbial cells of microorganism and dried product of the microbial cells.

24. The method of any one of claims 21 to 23, wherein the reaction is performed at temperature of 20 to 60° C and pH 4 to 9.

25. The method of any one of claims 21 to 23, wherein the reaction substrate is provided at 15 to 150 g/L.

26. The method of Claim 23, wherein the dried product of the microbial cells has 30% or more of a total conversion rate of Reb M and Reb D.

27. The method of Claim 23, wherein after the dried product of the microbial cells is stored at room temperature for 9 weeks, the enzyme activity of the microbial cells is 90% or higher of the Rebaudioside M conversion activity of the microbial cells immediately after drying.

28. The method of Claim 23, wherein the dried product of microbial cells are dried product that is freeze-dried at a temperature of -60 to 40°C and under reduced pressure conditions.

29. The method of Claim 23, wherein the reacting is performed with adding 1 to 20mM of ethylenediaminetetraacetic acid.

【FIG. 1】

【FIG. 2】

**INTERNATIONAL SEARCH REPORT**

| International application No. |
|---|
| **PCT/KR2022/021326** |

| A. | CLASSIFICATION OF SUBJECT MATTER |
|---|---|

**C12N 9/10**(2006.01)i; **C12P 19/56**(2006.01)i; **C12P 19/18**(2006.01)i; **C12N 15/62**(2006.01)i; **C12N 15/81**(2006.01)i

According to International Patent Classification (IPC) or to both national classification and IPC

| B. | FIELDS SEARCHED |
|---|---|

Minimum documentation searched (classification system followed by classification symbols)

C12N 9/10(2006.01); A23L 27/30(2016.01); C12N 15/70(2006.01); C12N 15/74(2006.01); C12P 19/18(2006.01); C12P 19/56(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 스테비올 배당체(steviol glycosides), 생산(production), 기질(substrate), 글루코스 (glucose), UDP-글리코실 전이효소(uridine diphosphate-glycosyltransferase), UGT76G1, 생촉매(biocatalyst), 레바우디오사 이드(rebaudioside)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | KR 10-2016-0111998 A (PEPSICO, INC.) 27 September 2016 (2016-09-27)<br>See abstract, claim 1, and paragraphs [0007] and [0060]-[0069]. | 1-29 |
| A | NCBI. Genbank accession no. XP_044372292.1 (25 October 2021).<br>See entire document. | 1-29 |
| A | KR 10-2015-0000258 A (KOREA RESEARCH INSTITUTE OF BIOSCIENCE AND BIOTECHNOLOGY) 02 January 2015 (2015-01-02)<br>See entire document. | 1-29 |
| A | KR 10-2016-0116775 A (AJOU UNIVERSITY INDUSTRY-ACADEMIC COOPERATION FOUNDATION) 10 October 2016 (2016-10-10)<br>See entire document. | 1-29 |
| A | US 2020-0087692 A1 (MANUS BIO, INC.) 19 March 2020 (2020-03-19)<br>See entire document. | 1-29 |

☑ Further documents are listed in the continuation of Box C.   ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **28 March 2023** | **29 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
|---|
| **PCT/KR2022/021326** |

**C.    DOCUMENTS CONSIDERED TO BE RELEVANT**

| Category* | Citation of document, with indication, where appropriate, of the relevant passages | Relevant to claim No. |
|---|---|---|
| A | KR 10-2019-0038612 A (AMYRIS, INC.) 08 April 2019 (2019-04-08)<br>See entire document. | 1-29 |

Form PCT/ISA/210 (second sheet) (July 2022)

**INTERNATIONAL SEARCH REPORT**

International application No.

**PCT/KR2022/021326**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

| International application No. |
| --- |
| **PCT/KR2022/021326** |

| Patent document cited in search report | | | Publication date (day/month/year) | Patent family member(s) | | | Publication date (day/month/year) |
| --- | --- | --- | --- | --- | --- | --- | --- |
| KR | 10-2016-0111998 | A | 27 September 2016 | CN | 106471128 | A | 01 March 2017 |
| | | | | EP | 3101139 | A1 | 07 December 2016 |
| | | | | EP | 3508584 | A1 | 10 July 2019 |
| | | | | JP | 2017-504341 | A | 09 February 2017 |
| | | | | JP | 6541159 | B2 | 10 July 2019 |
| | | | | KR | 10-2115640 | B1 | 26 May 2020 |
| | | | | US | 2017-0211113 | A1 | 27 July 2017 |
| | | | | WO | 2015-113231 | A1 | 06 August 2015 |
| KR | 10-2015-0000258 | A | 02 January 2015 | KR | 10-1559478 | B1 | 13 October 2015 |
| KR | 10-2016-0116775 | A | 10 October 2016 | KR | 10-1669041 | B1 | 25 October 2016 |
| US | 2020-0087692 | A1 | 19 March 2020 | CN | 112867799 | A | 28 May 2021 |
| | | | | EP | 3824093 | A2 | 26 May 2021 |
| | | | | EP | 3824093 | A4 | 01 June 2022 |
| | | | | JP | 2021-530228 | A | 11 November 2021 |
| | | | | KR | 10-2021-0066790 | A | 07 June 2021 |
| | | | | US | 11230724 | B2 | 25 January 2022 |
| | | | | US | 2022-0162658 | A1 | 26 May 2022 |
| | | | | WO | 2020-018506 | A2 | 23 January 2020 |
| | | | | WO | 2020-018506 | A3 | 12 March 2020 |
| KR | 10-2019-0038612 | A | 08 April 2019 | CN | 109804073 | A | 24 May 2019 |
| | | | | CN | 111263815 | A | 09 June 2020 |
| | | | | EP | 3497222 | A2 | 19 June 2019 |
| | | | | EP | 3497222 | B1 | 22 December 2021 |
| | | | | EP | 3665287 | A1 | 17 June 2020 |
| | | | | JP | 2019-524134 | A | 05 September 2019 |
| | | | | JP | 2020-533954 | A | 26 November 2020 |
| | | | | JP | 2022-137061 | A | 21 September 2022 |
| | | | | KR | 10-2020-0035981 | A | 06 April 2020 |
| | | | | KR | 10-2331018 | B1 | 26 November 2021 |
| | | | | US | 11091743 | B2 | 17 August 2021 |
| | | | | US | 2019-0185826 | A1 | 20 June 2019 |
| | | | | US | 2020-0165651 | A1 | 28 May 2020 |
| | | | | US | 2021-0355458 | A1 | 18 November 2021 |
| | | | | WO | 2018-031955 | A2 | 15 February 2018 |
| | | | | WO | 2018-031955 | A3 | 15 March 2018 |
| | | | | WO | 2019-033064 | A1 | 14 February 2019 |

Form PCT/ISA/210 (patent family annex) (July 2022)